# FASCICULE DE BREVET EUROPEEN

(11) **EP 1 531 332 B1**
(45) Date de publication et mention de la délivrance du brevet: **24.12.2008**
(21) Numéro de dépôt: 04292651.9
(22) Date de dépôt: 09.11.2004
(51) Int. Cl.: G01N 33/561, G01N 27/447, G01N 33/68, C07K 16/00

(54) **Analyse et typage de protéines monoclonales par électrophorèse capillaire et immunodéplacement**
Analyse und Typing von monoklonalen Proteinen mittels Kapillarelektrophorese und Immuno-Verschiebung
Analysis and typing of monoclonal proteins by capillary electrophoresis and immunoshift

(30) Priorité: 12.11.2003 FR 0313246
(43) Date de publication de la demande: 18.05.2005
(73) Titulaire: SEBIA, 91090 Lisses (FR)
(72) Inventeur: Robert, Frédéric, 91540 Mennecy (FR); André, Régis, 75013 Paris (FR)
(74) Mandataire: Desaix, Anne

(56) Documents cités:
- WO-A1-01/11003
- WO-A1-92/15316
- WO-A1-96/33412
- US-A- 5 567 282
- US-A- 5 985 275
- CLARK R ET AL: "DIFFERENTIAL DIAGNOSIS OF GAMMOPATHIES BY CAPILLARY ELECTROPHORESISAND IMMUNOSUBTRACTION: ANALYSIS OF SERUM SAMPLES PROBLEMATIC BY AGAROSE GEL ELECTROPHORESIS" ELECTROPHORESIS, WEINHEIM, DE, vol. 19, no. 14, octobre 1998 (1998-10), pages 2479-2484, XP001062511 ISSN: 0173-0835
- PAQUETTE DONALD M ET AL: "Detection of specific antibodies using immunosubtraction and capillary electrophoresis instrumentation" ELECTROPHORESIS, vol. 22, no. 12, août 2001 (2001-08), pages 2391-2397, XP002286281 ISSN: 0173-0835

## Description

L'invention concerne le domaine de l'analyse d'échantillons biologiques par électrophorèse capillaire et immunodéplacement.

L'invention a notamment pour objet une méthode d'analyse d'échantillons biologiques par électrophorèse capillaire dans laquelle la séparation électrophorétique des constituants de l'échantillon est accompagnée d'un traitement de l'échantillon par une technique immunologique. Dans le cadre de la présente demande, le terme « immunosoustraction » désigne la suppression du pic de certaines protéines dans le profil électrophorétique, par immunodéplacement de ce pic protéique à une autre position du profil.

L'invention a en particulier pour objet une méthode applicable dans des protocoles de diagnostic, et concerne notamment la recherche et le typage dans des échantillons biologiques de protéines témoins d'affections monoclonales, encore appelées gammapathies monoclonales ou paraprotéinémies.

Le développement normal des lymphocytes B conduit à la production, à la surface des lymphocytes B matures, d'immunoglobulines d'isotypes M et G, ayant initialement des idiotypes communs. Les désordres plasmocytaires qui sont à l'origine des affections monoclonales résultent d'un défaut de contrôle du processus de maturation cellulaire vers des cellules sécrétrices d'anticorps après exposition à un antigène spécifique de l'immunoglobuline de surface. Dans cette situation, des immunoglobulines ayant une affinité pour un antigène auquel l'hôte a été exposé, continuent d'être sécrétées après la disparition de l'antigène. De façon générale, les immunoglobulines d'une espèce donnée sont réparties en différentes classes d'anticorps, chaque classe étant identifiée à l'aide d'un isotype, les différents isotypes identifiés au sein d'une espèce étant communs à tous les individus normaux de cette espèce. Les immunoglobulines sont généralement formées de chaînes lourdes (2 chaînes lourdes) et de chaînes légères (2 chaînes légères). Sur la base de cette structure à quatre chaînes, on a identifié cinq isotypes de chaînes lourdes (isotypes M, G, A, D, E), et deux isotypes de chaînes légères (isotypes Kappa et Lambda). Outre leur isotype, les immunoglobulines sont caractérisées par des déterminants correspondant à des différences entre les individus d'une même espèce, appelés allotypes ainsi que par leur idiotype correspondant à une partie de la molécule d'immunoglobuline liant l'antigène. L'idiotype est donc caractéristique des molécules produites par un clone donné de cellules productrices d'anticorps.

Les maladies plasmocytaires sont donc notamment caractérisées par l'altération de la production de certaines immunoglobulines ou de certaines chaînes d'immunoglobulines, et la détection et la caractérisation de cette altération a un intérêt clinique manifeste.

L'analyse par électrophorèse d'un échantillon biologique permet l'identification des protéines sériques et la détermination de la quantité de ces protéines, en particulier des immunoglobulines. Dans un champ électrique, les protéines migrent en fonction de leur taille et de leur charge, formant un profil d'électrophorèse comportant une série de pics (également appelés fractions), chacun d'entre eux correspondant à une ou plusieurs protéines. La fraction appelée gamma est notamment formée d'immunoglobulines, principalement de type G. Chez des patients souffrant de maladies plasmocytaires conduisant à la sécrétion de protéines monoclonales (encore appelées protéines Mc), la quantité d'immunoglobulines correspondant à l'un des isotypes connus peut être augmentée de façon significative par rapport à la normale, conduisant à une modification du profil électrophorétique par la modification d'un ou plusieurs pics des protéines séparées d'un échantillon de sérum.

La détection des protéines monoclonales, en particulier la détection d'une augmentation de la production d'un isotype particulier d'immunoglobuline, présente donc un intérêt manifeste pour la recherche de maladies plasmocytaires mais aussi pour le suivi de patients atteints de paraprotéinémies. On a par exemple observé que la quantité d'une protéine Mc sérique peut selon les cas, notamment lors du développement tumoral, être directement reliée à la progression de l'affection. Ainsi, une protéine Mc peut constituer un marqueur tumoral qui, lorsqu'il est corrélé à d'autres symptômes, peut être pris en compte dans l'établissement d'un diagnostic.

Les maladies plasmocytaires ne sont pas seules concernées par la production anormale de protéines Mc ; parmi les désordres pathologiques associés à la production de protéines Mc, on citera les néoplasmes lymphoïdes, tels que la leucémie lymphoïde chronique ou les lymphomes d'origine lymphocytaire B ou T, certaines proliférations non lymphoïdes telles que la leucémie myéoloïde chronique, les cancers du sein ou du colon.

Des protéines Mc monoclonales peuvent aussi être produites dans certaines pathologies non malignes telles que la cyrrhose, la sarcoïdose, des parasitoses ou la maladie de Gaucher. On détecte encore la production de protéines monoclonales dans des affections auto-immunes, telles que la polyarthrite rhumatoïde, la myasthénie, ou la maladie des agglutinines froides.

L'électrophorèse sur gel d'agarose et l'électrophorèse capillaire, en permettant de détecter la présence de protéines monoclonales dans des échantillons biologiques et de suivre dans le temps l'évolution des protéines monoclonales détectées, constituent donc des méthodes de choix en vue de l'établissement d'un diagnostic ou du suivi de patients.

Selon les pathologies concernées, la protéine Mc est de nature différente, constituée soit d'une molécule d'anticorps intacte, soit d'un fragment d'anticorps. Ainsi, des chaînes lourdes ou des chaînes légères peuvent être produites seules. C'est le cas par exemple des protéines de Bence Jones, sécrétées dans les urines des patients atteints de myélomes et se présentant sous la forme des seules chaînes légères.

Les isotypes déterminés des immunoglobulines permettent de typer les protéines Mc, en fonction de la nature de leur chaîne lourde et/ou en fonction de la nature de leur chaîne légère. La technique utilisée pour le typage des immunoglobulines permettra ainsi de déterminer le type de chaînes lourde et/ou légère associées de chaque protéine monoclonale présente dans un échantillon biologique.

Outre la détection de la présence de ces protéines Mc, il est donc apparu important de les typer, pour permettre de caractériser la pathologie qui leur est associée. Pour ce faire, différentes approches ont déjà été proposées, telles que la chromatographie sur colonne, l'électrophorèse sur gel d'agarose ou encore l'électrophorèse capillaire. Dans des méthodes mettant en oeuvre l'électrophorèse sur gel d'agarose, les protéines de l'échantillon biologique sont séparées par électrophorèse sous la forme d'un profil d'électrophorèse dans lequel les protéines et en particulier les globulines, sont révélées sous forme de pics ou bandes susceptibles d'inclure une protéine monoclonale, dont la nature doit ensuite être confirmée, par exemple par la technique d'immunofixation sur gel d'agarose. Cette technique combine deux étapes en utilisant une électrophorèse sur gel d'agarose puis une immuno-précipitation. Plusieurs aliquotes d'un même échantillon biologique sont déposées en parallèle sur un gel d'agarose, puis l'application d'un champ électrique permet la séparation des protéines, et notamment des immunoglobulines. Chaque piste est ensuite incubée avec un type d'anticorps spécifiques des types d'immunoglobulines recherchées (IgG, IgA, IgM, kappa et lambda, et éventuellement kappa libre, lambda libre, IgD et IgE), conduisant à la formation d'immuncomplexes entre les immunoglobulines de l'échantillon et les anticorps. Après un lavage du gel qui permet d'éliminer les protéines n'ayant pas précipité, une étape de coloration permet de révéler la position des immuncomplexes : en cas d'absence de protéines monoclonales, seul un fond coloré diffus apparaît (correspondant à la multitude d'anticorps monoclonaux constituant le « fond polyclonal ») ; en cas de présence de protéines monoclonales, des bandes colorées sont révélées dans des régions spécifiques du gel. En utilisant une piste de référence (sans antisérum), on peut ainsi typer chaque bande monoclonale visible sur le gel.

Une technique de typage utilisée en électrophorèse capillaire est l'immunosoustraction telle que décrite dans le brevet US 5,228,960. Cette technique consiste à incuber des aliquotes d'un échantillon biologique avec des anticorps spécifiques capables de soustraire un constituant donné (par exemple des immunoglobulines) dudit échantillon. Ce constituant reste adsorbé sur la phase solide sur laquelle a été fixé l'anticorps : il n'est donc plus présent dans l'échantillon analysé par électrophorèse capillaire. Les différentes aliquotes traitées dans lesquelles ont été soustraites certaines immunoglobulines selon la spécificité des anticorps utilisés sont alors injectées dans le capillaire, puis les protéines contenues dans les aliquotes sont séparées par application d'un champ électrique aux bornes du capillaire. Les profils obtenus sont comparés à un profil d'une aliquote non traitée.

D'autres techniques d'identification des protéines monoclonales, séparées par électrophorèse capillaire à partir d'un échantillon, ont été proposées, par exemple dans le brevet EP 0 690 988 (B1). Le brevet européen en question décrit l'association à la séparation électrophorétique capillaire, d'une étape d'immunosoustraction, dans le but de faciliter la classification des protéines Mc. Selon ce brevet, l'immunosoustraction est réalisée « on - capillaire » selon une méthode qui comprend une étape de séparation électrophorétique d'une première partie de l'échantillon en ses différents constituants, et la détection de ces constituants, puis le mélange d'une seconde partie de l'échantillon avec au moins un partenaire capable de lier spécifiquement un analyte prédéterminé contenu dans l'échantillon, étant entendu que le partenaire spécifique pour cette liaison a une mobilité électrophorétique différente de celle dudit analyte. La seconde partie de l'échantillon est ensuite séparée en ses différents constituants par électrophorèse capillaire et les constituants sont détectés. Une étape de comparaison des constituants séparés est ensuite réalisée avec les constituants séparés hors de la présence des partenaires spécifiques liant un analyte recherché.

Le brevet européen EP 0 690 988 précise que le partenaire destiné à lier les analytes est une molécule modifiée, en particulier un anticorps anti-protéine Mc, qui a subi une modification chimique de façon à ce que son temps de migration en électrophorèse capillaire, le conduise hors de la région Gamma du profil électrophorétique. Une modification chimique proposée des anticorps est celle qui consiste à les faire réagir avec l'anhydride succinique pour leur apporter des fonctions carboxyliques supplémentaires, négatives à pH alcalin. Dans les conditions d'analyses décrites (pH 10), la charge négative globale des anticorps est donc augmentée.

Les conditions décrites pour réaliser la modification des anticorps, selon le brevet européen EP 0 690 988, ainsi que les résultats expérimentaux rapportés, qui concernent uniquement l'analyse et la séparation d'immunoglobulines G purifiées et non pas l'analyse d'un sérum, permettent de douter de la pertinence de la méthode proposée dans le but de rechercher des protéines monoclonales dans un échantillon biologique « réel », par exemple dans un sérum.

WO 96/33412 décrit des compositions, procédés et dispositifs pour réaliser des dosages ultrarapides par liaison, de préférence par électrophorèse capillaire. Dans un mode de réalisation, un premier partenaire de liaison porte une étiquette détectable et un second partenaire de liaison (p.ex. un anticorps) est modifié pour être fortement chargé.

Des exemples de composés chargés sont donnés. Certains d'entre eux apportent une ou plusieurs charges négatives pour chaque fonction chimique modifiée de l'anticorps. Il y a parmi eux des polymères possédant plusieurs fonctions acides carboxyliques et étant liés par un agent de couplage.

La combinaison d'un échantillon contenant un analyte avec lequel les deux partenaires de liaison peuvent interagir et sur lequel ils peuvent se fixer provoque la formation d'un complexe à trois éléments. La différence de mobilité électrophorétique entre les formes libre et liée au complexe du premier partenaire de liaison marqué est telle qu'elle permet l'électroséparation et la détection ultérieure d'un analyte. Les compositions, procédés et dispositifs décrits dans ce document permettent de déterminer quantitativement la concentration d'un analyte dans un échantillon. Cet analyte peut être un anticorps monoclonal. Des kits / trousses sont aussi décrits.

La présente invention propose une méthode alternative applicable efficacement sur un échantillon biologique, associant la réalisation d'une électrophorèse capillaire pour la séparation des constituants d'un échantillon biologique et l'immunosoustraction pour permettre le typage des protéines Mc éventuellement présentes dans l'échantillon biologique analysé.

L'un des avantages de la méthode de l'invention est de permettre un déplacement, hors de la zone correspondant au profil de migration des protéines de l'échantillon, en particulier hors de la zone de migration des globulines, du pic d'électrophorèse correspondant à une protéine Mc lorsqu'elle est présente dans un échantillon biologique et qu'elle a été séparée par électrophorèse capillaire. Ce déplacement soustrait le pic représentant la protéine Mc séparée, de sa position attendue à l'issue de l'étape de migration sans interférer avec la séparation des autres protéines de l'échantillon. Contrairement à la méthode décrite dans le brevet US 5,228,960, la protéine monoclonale reconnue par l'anticorps est présente dans l'échantillon injecté pour réaliser l'électrophorèse capillaire. Elle est simplement soustraite du profil car déplacée de sa position initiale. En conséquence, cette méthode permet une lecture fiable des résultats de l'analyse, dissipant toute éventuelle confusion entre des pics voisins correspondant aux protéines séparées de l'échantillon.

L'invention concerne également des anticorps modifiés chimiquement pour leur apporter des fonctions chimiques, supplémentaires, négatives à pH alcalin (ici des fonctions carboxyliques), par réaction de certaines fonctions chimiques de l'anticorps avec un agent modificateur : chacune des fonctions chimiques modifiées apporte ainsi plusieurs charges négatives supplémentaires à l'anticorps. Ces anticorps sont ensuite utilisés pour l'immunosoustraction/immunodéplacement spécifique des pics correspondant à des protéines Mc d'un échantillon biologique.

Les anticorps ainsi préparés dans le cadre de l'invention, présentent par rapport aux anticorps de départ déjà chargés négativement lorsqu'ils sont à pH alcalin, un supplément de charges négatives lorsqu'ils sont à pH alcalin, du fait de l'acquisition de fonctions telles que des fonctions acides carboxyliques supplémentaires. Ces anticorps sont désignés dans la suite par l'expression « anticorps modifiés surchargés négativement » ou aussi par les termes « anticorps modifiés ».

L'invention a encore pour objet des kits pour la réalisation d'une séparation des protéines d'un échantillon biologique par électrophorèse capillaire et la détection par immunosoustraction afin de les identifier et, le cas échéant, de les quantifier, des protéines monoclonales susceptibles d'être contenues dans l'échantillon biologique testé.

L'invention concerne aussi une méthode de préparation d'anticorps modifiés chimiquement pour leur greffer des charges négatives supplémentaires à pH alcalin (anticorps modifiés surchargés négativement), dans des conditions permettant leur utilisation dans une immunosoustraction.

L'invention concerne donc une méthode pour l'analyse électrophorétique capillaire d'un échantillon biologique, comprenant la mise en ceuvre d'anticorps modifiés surchargés négativement de façon telle qu'ils migrent en une zone située en dehors de la zone de migration des protéines de l'échantillon biologique lorsqu'elles sont séparées lors de l'électrophorèse, lesdits anticorps ayant une spécificité antigénique pour une protéine monoclonale déterminée.

L'invention est définie par les revendications.

Lors de la séparation des protéines d'un échantillon biologique, dans un capillaire rempli d'un électrolyte, les protéines migrent sous l'effet d'un champ électrique : les protéines sont ainsi entraînées dans une migration de l'anode vers la cathode. La différence entre vitesse électrophorétique propre (liée à la charge de la protéine) et flux électroosmotique (lié à la charge de la surface interne du capillaire) permet la séparation des protéines à détecter.

Les protéines d'un échantillon injecté dans le capillaire, définissent donc un profil de migration électrophorétique que l'on peut découper en plusieurs zones, illustrées sur les figures de la présente demande, et correspondant aux zones gamma (la plus proche de la cathode), beta2, beta1, alpha2, alpha1 et albumine (cette dernière zone étant la plus proche de l'anode). Les immunoglobulines contenues dans un échantillon biologique migrent de la zone alpha1 à la zone gamma, y compris les protéines Mc.

Dans le but notamment de permettre la séparation électrophorétique de protéines monoclonales contenues dans un échantillon biologique ainsi que leur typage fiable, au moyen d'une réaction d'immunosoustraction, les inventeurs ont mis au point des conditions de réalisation de l'immunosoustraction qui permettent la soustraction du pic correspondant à une protéine monoclonale, du profil de migration des protéines de l'échantillon (et en particulier du profil de migration des globulines) et son déplacement en dehors de la région comprise entre de la zone gamma à la zone alpha1.

Plus précisément, la protéine monoclonale recherchée dans l'échantillon n'est pas soustraite du milieu analysé mais déplacée sous l'effet de sa liaison avec un anticorps modifié surchargé négativement la reconnaissant spécifiquement, dans des conditions telles que le complexe formé entre ladite protéine monoclonale et ledit anticorps modifié est déplacé en dehors du profil de migration des globulines de l'échantillon ( Les globulines correspondent aux fractions électrophorétiques gammas, bêta-2, bêta-1 , alpha-2 et alpha-1 sur le profil électrophorétique).

Plus précisément encore, le complexe protéine Mc-anticorps chargé négativement à pH alcalin, se trouve après l'étape de migration électrophorétique, dans la zone la plus anodique du profil des protéines séparées.

Le complexe anticorps modifié -protéine Mc est donc visible en dehors du profil ainsi défini par les immunoglobulines séparées, notamment est éloigné de la zone gamma vers l'albumine, étant éventuellement visible au voisinage de l'albumine et d'alpha-1, sans que cela gêne l'éventuelle détection d'une protéine monoclonale anodique (c'est à dire migrant en alpha-1/alpha-2) puisque le complexe a une mobilité intermédiaire entre celle de la protéine monoclonale et celle de l'anticorps modifié.

L'anticorps modifié est en principe apporté en excès pour la réaction et la fraction d'anticorps n'ayant pas réagi est visible vers l'anode au-delà du pic correspondant à l'albumine (c'est à dire de migration plus anodique que l'albumine).

Une première méthode selon l'invention pour l'analyse électrophorétique capillaire d'un échantillon biologique et l'immunosoustraction, comprend les étapes suivantes :
a) séparation des constituants d'une première partie aliquote de l'échantillon biologique par électrophorèse capillaire, en présence d'anticorps modifiés surchargés négativement ayant une spécificité antigénique déterminée et susceptibles de former un complexe de type antigène-anticorps avec une protéine monoclonale éventuellement présente dans l'échantillon biologique et détection des constituants séparés de l'échantillon biologique ;
b) comparaison du profil électrophorétique obtenu à l'étape a) avec un profil électrophorétique des constituants d'une autre partie aliquote du même échantillon séparé par électrophorèse capillaire en l'absence desdits anticorps modifiés surchargés négativement de spécificité antigénique déterminée.

On observera que les échantillons biologiques comportent normalement une fraction d'immunoglobulines polyclonales constituant un « fond polyclonal » situé dans la région gamma (ou proche de cette dernière) du profil électrophorétique. Ces immunoglobulines polyclonales apparaissent sous la forme d'un pic plus large que celui des immunoglobulines Mc (car contenant un grand nombre d'immunoglobulines de mobilités très proches), dans les conditions de réalisation d'une électrophorèse capillaire propres à la séparation de protéines Mc ; ce fond polyclonal peut être diminué lors de la mise en contact de l'échantillon avec les anticorps modifiés surchargés négativement, selon le type d'immunoglobulines le constituant (G, A, M, kappa ou lambda). La distinction entre pic monoclonal et fond polyclonal se fait dans ce cas en repérant le pic Mc focalisé à détecter par rapport au pic large polyclonal.

Dans un mode de réalisation préféré de l'invention, on réalise parallèlement la mise en contact de parties aliquotes d'un échantillon biologique donné avec différents anticorps, formant une gamme d'anticorps modifiés surchargés négativement, chaque type d'anticorps de cette gamme ayant une spécificité antigénique déterminée pour un isotype d'immunoglobulines.

Ainsi, on peut réaliser parallèlement l'analyse d'un même échantillon biologique avec lesdits anticorps modifiés de spécificité différente, la spécificité antigénique de ces anticorps étant choisie en fonction des protéines monoclonales recherchées qui seraient reconnues par ces anticorps et en fonction du nombre de capillaires en parallèle sur l'appareil d'électrophorèse.

Selon encore un mode de réalisation particulier de l'invention, dans l'une des méthodes décrites ci-dessus, l'échantillon biologique est divisé en au moins deux parties aliquotes et la méthode comprend les étapes suivantes, préalables à la séparation des constituants de l'échantillon biologique par électrophorèse :
1) Mise en contact d'un milieu contenant des anticorps modifiés surchargés négativement ayant une spécificité antigénique déterminée avec une partie aliquote de l'échantillon biologique, dans des conditions d'incubation permettant la réaction immunologique entre lesdits anticorps et une protéine cible spécifiquement reconnue par lesdits anticorps, lorsqu'elle est présente dans l'échantillon biologique, et mise en contact d'une autre partie aliquote de l'échantillon biologique avec le susdit milieu cependant dépourvu des anticorps modifiés,
2) injection dans le capillaire d'électrophorèse, des parties aliquotes de l'échantillon biologique incubées selon l'étape 1).

Dans un autre mode de réalisation particulier de l'invention, la méthode d'analyse électrophorétique capillaire comprend les étapes suivantes, préalables à la séparation des constituants de l'échantillon biologique par électrophorèse :
1) injection d'un milieu contenant des anticorps modifiés surchargés négativement ayant une spécificité antigénique déterminée, dans le capillaire d'électrophorèse, et injection dudit milieu cependant dépourvu desdits anticorps modifiés dans un autre capillaire d'électrophorèse ;
2) injection de l'échantillon biologique dans les capillaires d'électrophorèse traités selon l'étape 1), dans des conditions permettant la réaction immunologique entre les anticorps et la ou les protéine(s) cible(s) lorsqu'elle(s) est(sont) présente(s) dans l'échantillon biologique.

Pour l'analyse d'un échantillon biologique, lorsque la gamme d'anticorps modifiés surchargés négativement comprend n types d'anticorps de spécificité différente déterminée, on séparera l'échantillon biologique en au moins n + 1 parties aliquotes.

Les anticorps utilisés pour préparer les anticorps modifiés surchargés négativement sont des immunoglobulines choisies parmi des anticorps anti-IgG, anti-IgA, anti-IgM, anti-IgE, anti-IgD, anti-Kappa, anti-Lambda, anti-kappa libre et anti-lambda libre.

De façon préférée, les anticorps sont choisis parmi les anticorps anti-IgG, anti-IgA, anti-IgM, anti-Kappa et anti-Lambda. Selon un mode de réalisation particulier de l'invention, on utilise une gamme d'anticorps comprenant ces 5 anticorps. Selon un autre mode de réalisation de l'invention, on utilise un mélange de tout ou partie de ces anticorps, par exemple un mélange de 5 anticorps (antisérum pentavalent) ou un mélange de 3 anticorps (antisérum trivalent) par exemple contenant des anticorps anti-chaînes lourdes IgG, IgA, IgM.

On peut aussi limiter la mise en ceuvre de la méthode de l'invention à l'utilisation de seulement certains des anticorps ci-dessus identifiés.

Pour obtenir des anticorps surchargés négativement, on réalise une réaction des anticorps ayant la spécificité antigénique choisie avec un composé apportant des charges supplémentaires négatives à pH alcalin, par exemple un anhydride chargé négativement. On fait par exemple réagir des fonctions amines des anticorps avec un anhydride, d'où la formation d'une fonction acide carboxylique supplémentaire sur l'anticorps (et donc d'une charge négative supplémentaire à pH alcalin) pour chaque fonction amine modifiée.

Dans le cadre de la présente invention, l'anhydride comprend au moins une fonction acide carboxylique.

Pour la réalisation de ces anticorps modifiés surchargés négativement, l'anhydride mis en oeuvre est avantageusement un anhydride benzène tricarboxylique. Cet anhydride apporte deux fonctions acide carboxylique (et donc deux charges négatives supplémentaires à pH alcalin) pour chaque fonction amine modifiée : une première fonction grâce à la réaction de l'anhydride avec la fonction amine, une seconde fonction de par sa structure. L'anhydride sera ainsi appelé anhydride chargé négativement, puisque porteur d'au moins une fonction acide carboxylique chargée négativement à pH alcalin.

On peut obtenir un anticorps suffisamment modifié donc suffisamment chargé négativement à pH alcalin en effectuant la réaction de l'anticorps avec l'anhydride chargé négativement, par dissolution extemporanée de l'anhydride, notamment de l'anhydride benzène tricarboxylique, dans du diméthylformamide . (DMF) et la mise en contact de l'anhydride dissous avec l'anticorps en solution pour permettre l'apport de charges négatives à l'anticorps, ladite réaction étant effectuée à 37°C dans des conditions permettant le greffage de fonctions acide carboxylique sur l'anticorps.

Le greffage de l'anhydride sur l'anticorps se fait avec d'autant plus d'efficacité que la solution d'anhydride dans le DMF est mise au dernier moment au contact avec l'anticorps pour obtenir des anticorps avec des fonctions acide carboxylique supplémentaires (et donc des charges négatives supplémentaires à pH alcalin) et ce de façon à prévenir l'altération de l'anhydride avec l'eau contenue dans la solution d'anticorps.

Une méthode de préparation des anticorps modifiés est décrite avec plus de détails dans la suite de la demande.

Pour apporter des fonctions acide carboxylique supplémentaires (et donc des charges négatives supplémentaires à pH alcalin) à l'anticorps, on peut en variante utiliser l'acide mellitique (apporteur des charges négatives à pH alcalin) auquel on associe l'agent de couplage EDCI ou 1-(3-diméthylaminopropyl)-3-ethylcarbodiimide hydrochloride). L'acide mellitique réagit avec les fonctions amines des anticorps en présence de l'EDCI.

Les anticorps modifiés mis en oeuvre dans la méthode d'analyse électrophorétique capillaire de l'invention sont normalement en excès par rapport à la protéine cible qu'ils reconnaissent lorsqu'elle est présente dans l'échantillon biologique.

Cet excès permet non seulement une disparition totale de la protéine Mc cible du profil protéique, mais aussi une position optimale du complexe anticorps modifié - protéine Mc sur le profil : un rapport trop faible entre les anticorps modifiés ayant une spécificité antigénique déterminée et les protéines cibles de l'échantillon reconnues par cet anticorps ne permettrait pas une soustraction totale du pic Mc et donnerait une migration plus cathodique du complexe que lorsque ce rapport est plus élevé.

Dans un mode de réalisation particulier de l'invention, la concentration d'anticorps chargés négativement de chaque spécificité mis en oeuvre pour l'immunosoustraction est d'environ 10 g/l.

L'invention a aussi pour objet des anticorps modifiés surchargés négativement, qui sont le produit de la réaction d'anticorps avec un anhydride apporteur d'au moins 2 fonctions acide carboxylique, de préférence l'anhydride 1,2,4-benzène tricarboxylique.

Les anticorps mis en ceuvre dans la méthode d'analyse par électrophorèse capillaire et immunosoustraction, sont ainsi avantageusement le produit de la réaction de l'anticorps avec un anhydride chargé négativement, ladite réaction comprenant la dissolution extemporanée de l'anhydride dans du diméthylformamide (DMF) et la mise en contact de l'anhydride dissous avec l'anticorps en solution pour permettre la réaction de l'anhydride et de l'anticorps, ladite réaction étant effectuée à 37°C dans des conditions permettant le greffage de fonctions chimiques supplémentaires sur l'anticorps, notamment des fonctions acides carboxyliques, négatives à pH alcalin.

La méthode d'analyse d'échantillons biologiques peut être mise en oeuvre sur différents types d'échantillons biologiques, tels que des échantillons de sérum, de plasma, d'urine ou de liquide céphalo-rachidien.

Selon la nature de l'échantillon biologique utilisé et/ou son origine (c'est à dire selon le dossier médical du patient), l'interprétation des résultats issus de la séparation électrophorétique doit éventuellement tenir compte d'interférences pouvant se produire entre les anticorps surchargés négativement et les protéines de l'échantillon. De telles interférences sont perceptibles et analysables par l'homme du métier et ont été observées pour les échantillons contenant du fibrinogène, à savoir les échantillons de plasma ou les sérums de certains patients traités avec des composés anticoagulants. Les inventeurs ont observé que le fribinogène peut réagir, par une réaction non spécifique, i.e., une réaction qui n'est pas du type antigène-anticorps, avec les anticorps modifiés surchargés négativement de l'invention. Dans ce cas, lorsque du fibrinogène est présent, ce qui se traduit sur un profil électrophorétique par la présence d'un pic entre les zones β et γ, ce pic est supprimé de sa zone normale de migration après que l'échantillon ait été traité avec les anticorps surchargés négativement de l'invention, cette suppression se manifestant quelle que soit la spécificité des anticorps surchargés négativement mis en oeuvre. En d'autres termes, le pic marquant la présence de fibrinogène dans l'échantillon est révélé uniquement dans le profil électrophorétique témoin (ELP) et disparaît du profil des protéines soumises aux réactions immunologiques avec les anticorps modifiés surchargés négativement (par immunodéplacement dans une zone en dehors de ce profil).

Les conditions de réalisation de l'électrophorèse capillaire en veine liquide sont les conditions habituellement mises en oeuvre par l'homme du métier pour les étapes d'injection de l'échantillon dans le tube capillaire, de séparation des constituants de l'échantillon par migration électrophorétique sous l'effet d'un courant d'électroendosmose. Ces étapes comportent notamment l'utilisation de tampons d'électrophorèse tels que ceux décrits dans les demandes de brevets WO 02/057736 et WO 02/057737 étant entendu que les tampons d'électrophorèse sont choisis pour permettre la séparation de protéines Mc sous la forme de pics. Des conditions de réalisation d'une électrophorèse capillaire sont par exemple les conditions appropriées pour utiliser l'automate Capillarys (SEBIA), y compris les tampons vendus sous la dénomination SEBIA CAPILLARYS B1 B2 + et CAPILLARYS PROTEIN(E) 6 et CAPILLARYS PROTEIN(E) 5.

Par exemple, le tampon d'analyse utilisé pour la migration électrophorétique est avantageusement un tampon alcalin, dont le pH est compris dans l'intervalle 9 à 11, de préférence autour de 10, comprenant en particulier un composé tampon et au moins un additif susceptible d'augmenter la force ionique du tampon d'analyse.

La méthode d'analyse électrophorétique capillaire décrite ci-dessus permet, par l'effet de la modification chimique de la mobilité des anticorps utilisés pour réaliser l'étape d'immunosoustraction, de remédier aux conséquences qui pourraient résulter de la co-migration des protéines monoclonales avec les constituants protéiques de l'échantillon lors de l'étape de séparation électrophorétique. Ainsi, la méthode de l'invention permet d'éviter les interférences entre les protéines monoclonales reconnues par les anticorps modifiés surchargés négativement et les autres protéines constituant l'échantillon biologique. Cet avantage essentiel pour la détection fiable des protéines monoclonales d'un échantillon biologique, résulte notamment du fait que le complexe protéine Mc-anticorps modifié, migre après les globulines et donc permet le typage de la protéine Mc reconnue par l'anticorps modifié surchargé négativement quelle que soit la position de la protéine monoclonale lors de la migration électrophorétique.

En outre, l'immunosoustraction du pic correspondant au complexe protéine Mc - anticorps chargé négativement est totale dans le sens où le pic qui correspondrait à la protéine monoclonale n'est plus détectable dans la zone correspondant au profil de migration des globulines de l'échantillon biologique.

En conséquence, la méthode de l'invention est avantageusement applicable à la détection et au typage de tous les types d'immunoglobulines monoclonales IgG, IgA, IgM, IgD, IgE et chaînes libres quelle que soit leur mobilité dans la mesure où les zones gammas, bêtas et alphas du profil de migration sont sans interférence avec la zone de migration de l'anticorps modifié surchargé négativement et du complexe qu'il forme avec les protéines Mc lorsqu'elles sont présentes dans l'échantillon biologique.

La méthode de l'invention est donc applicable pour l'analyse électrophorétique capillaire de protéines monoclonales dans un échantillon biologique.

Pour la détection et le typage de protéines monoclonales dans un échantillon biologique, on effectue avantageusement plusieurs analyses capillaires en parallèle, l'échantillon biologique étant divisé en différentes parties aliquotes, et au moins en un nombre de parties aliquotes égal ou supérieur au nombre de types d'immunoglobulines utilisés pour la recherche des protéines Mc.

Pour la réalisation du typage des protéines Mc dans un échantillon biologique, on réalisera normalement au moins 6 analyses capillaires parallèles.

Avantageusement, les anticorps modifiés surchargés négativement sont des immunoglobulines dont la spécificité antigénique est choisie parmi les immunoglobulines anti-IgG, anti-IgA, anti-IgM, anti-kappa et anti-lambda et/ou anti-kappa libre, et/ou anti-lambda libre.

Peuvent s'y ajouter des immunoglobulines anti-IgD, ou/et anti-IgE.

Outre le fait que la protéine Mc éventuellement présente dans l'échantillon biologique peut être typée par la méthode de l'invention, la dite protéine Mc, lorsqu'elle est présente, peut être quantifiée. Pour ce faire, on détermine la surface du pic correspondant à la protéine Mc ayant été soustraite du profil.

La méthode de l'invention peut également être utilisée pour identifier d'autres protéines (hors immunoglobulines) visibles sur le profil électrophorétique ; on pourra par exemple utiliser des anticorps modifiés de spécificité anti-human-haptoglobine, -alpha 1 antitrypsine, -complément C3, -complément C4, -transferrine, -RBP, β2-microglobuline, -α1-microglobuline, ou des anticorps d'autres spécificités. Grâce à ces anticorps, il serait ainsi possible d'identifier les protéines constituant une fraction d'un profil électrophorétique, ou bien de repérer un phénotype particulier de ces protéines ayant une mobilité différente de la normale.

Les conditions de réalisation de l'invention décrites dans la présente demande, en vue de détecter des protéines autres qu'immunoglobulines, s'appliquent par analogie.

L'invention a par ailleurs pour objet un procédé pour la préparation d'anticorps modifiés surchargés négativement, comprenant les étapes de :
- dissolution extemporanée d'un anhydride comportant au moins une fonction acide carboxylique dans du diméthylformamide (DMF),
- mise en contact des anticorps avec l'anhydride dissous dans des conditions permettant la réaction de l'anhydride et de l'anticorps afin d'obtenir un anticorps modifié surchargé négativement à pH alcalin.

L'anhydride choisi pour apporter des charges supplémentaires négatives à pH alcalin à l'anticorps est un anhydride possédant au moins une fonction acide carboxylique.

L'anhydride choisi pour apporter des charges supplémentaires négatives à pH alcalin à l'anticorps est de préférence l'anhydride benzène tricarboxylique.

La préparation des anticorps modifiés surchargés négativement est décrite en détail dans la partie expérimentale.

On souligne que pour être efficace s'agissant de l'apport de charges négatives, les étapes précédemment définies comportent avantageusement la dissolution de l'anhydride dans du diméthylformamide pur, puis le mélange de cette solution d'anhydride au moment du greffage avec l'anticorps, c'est à dire tardivement.

Le respect de ces conditions permet d'éviter une destruction de l'anhydride par l'eau contenue dans la solution d'anticorps, car ladite destruction altérerait l'efficacité de la préparation des anticorps chargés en diminuant l'apport de charges négatives à pH alcalin.

De façon préférée, la réaction entre l'anhydride et l'anticorps, notamment entre l'anhydride benzène tricarboxylique et l'anticorps est effectuée à une température de 37°C, ou à une température appropriée permettant d'augmenter le nombre de molécules d'anhydrides greffées sur l'anticorps, par exemple pendant une durée d'environ une heure.

Avant d'être mis au contact avec l'anhydride, l'antisérum à greffer est avantageusement dialysé contre une solution de tampon PBS ou de tampon phosphate. En outre, lorsque l'anticorps est mis en contact avec la solution d'anhydride dans le DMF, on ajoute de préférence de la soude, ce qui permet de mieux tamponner les fonctions acide carboxylique de l'anhydride benzène tricarboxylique déjà présentes ainsi que celles qui se forment au fur et à mesure de la réaction de l'anhydride avec l'anticorps (de façon à conserver un pH neutre au milieu réactionnel ce qui permet à la réaction de l'anhydride avec les fonctions amines de l'anticorps de se poursuivre).

Selon un mode de réalisation particulièrement préféré de l'invention, lors de la mise en contact avec l'anhydride dans le DMF, l'anticorps préalablement dialysé contre un tampon PBS ou un tampon phosphate à pH 7,4 est mis en contact avec de la soude 5N, et on ajoute de préférence autant d'équivalents de soude que de fonctions acide carboxylique (1) formées lors de la réaction et (2) amenées par l'anhydride. Les anticorps modifiés sont ensuite dialysés dans un milieu permettant la conservation des anticorps, puis ces solutions sont ramenées à la concentration adéquate de travail (environ 10 g/l) afin de se placer dans des conditions où le rapport anticorps/antigène sera optimal.

L'invention a aussi pour objet des anticorps modifiés surchargés négativement, caractérisés en ce qu'ils comportent des fonctions acide carboxylique greffées de façon à leur conférer une capacité de migration électrophorétique en dehors du profil de migration des immunoglobulines d'un échantillon biologique séparées dans les mêmes conditions de séparation par électrophorèse capillaire.

Les anticorps chargés négativement peuvent être des anticorps monoclonaux. Il peut alternativement s'agir d'anticorps polyclonaux.

Le contact entre l'anticorps et les protéines antigéniques de l'échantillon peut être effectué de deux manières : un mélange de la solution d'anticorps modifié avec l'échantillon biologique, de préférence dilué, dans le support d'échantillons, par exemple, dans la barrette Capillarys, préalablement à son injection dans le capillaire, ou alternativement les injections successives de la solution d'anticorps modifié puis de l'échantillon dilué dans le capillaire. Ce second mode de réalisation de l'étape de mise en contact permet une réaction antigène/anticorps à l'intérieur du capillaire (« in-capillaire ») tout aussi efficace que le mode classique de mélange des 2 solutions dans la barrette ou autre support d'échantillons, et est rendu possible du fait des différences de mobilité des anticorps modifiés par rapport aux protéines de l'échantillon et de l'ordre d'injection des 2 solutions : les anticorps modifiés ayant une mobilité beaucoup plus faible que les protéines de l'échantillon (du fait de leur charge négative importante) et étant injectés en premier, l'injection de l'échantillon après celle des anticorps modifiés provoque le passage de l'échantillon à travers la zone d'anticorps, et permet donc la soustraction du profil protéique des antigènes reconnus par les anticorps.

L'invention concerne aussi un kit de détection de protéines monoclonales dans un échantillon biologique, comprenant des anticorps selon l'invention et d'autres composants, par exemple le milieu destiné à contenir les anticorps, toutefois sans les anticorps (permettant d'obtenir le profil de référence) et/ou des tampons d'électrophorèse et/ou des solutions de lavage des capillaires et/ou des barrettes de dilution spécifiques à volumes réduits et/ou un diluant de l'échantillon à analyser, pemettant par exemple une bonne superposition des différentes courbes obtenues lors des différentes analyses. Ce kit comprend éventuellement des indications d'utilisation pour réaliser l'analyse électrophorétique et l'immunodéplacement de façon à révéler et typer les protéines Mc.

De façon préférée, ce kit contient une gamme d'anticorps de spécificité anti-IgG, anti-IgA, anti-IgM, anti-kappa et anti-lambda.

Un autre type de kit peut contenir un mélange d'anticorps de spécificité anti-IgG, anti-IgA, anti-IgM, anti-kappa et anti-lambda. Ce mélange, que l'on peut nommer antisérum Pentavalent, permet de confirmer la nature monoclonale d'un pic apparu sur le profil électrophorétique ou d'une déformation d'une fraction de ce profil. Ce kit est particulièrement intéressant puisque les immunoglobulines Mc peuvent apparaître non seulement dans la zone gamma, mais également dans les zones bêta et alpha, et peuvent éventuellement être masquées par les protéines habituelles de ces zones sur le profil obtenu en électrophorèse capillaire. Dans ce mode de réalisation de l'invention, l'échantillon est testé sur 2 pistes : une piste de référence (contenant seulement le milieu des anticorps, sans les anticorps) et une piste permettant de révéler la présence d'un pic monoclonal sur le profil électrophorétique (à l'aide de l'antisérum Pentavalent).

Un autre type de kit peut contenir un mélange d'anticorps de spécificité anti-chaînes lourdes (γ, α, et µ, anti-IgG, anti-IgA, anti-IgM que l'on peut appeler antisérum Trivalent), et/ou des anticorps de spécificités anti-chaînes légères kappa et lambda (libres et liées), et/ou des anticorps de spécificité anti-chaînes légères libres kappa et lambda. Ce kit permettrait la détection et l'identification de protéines de Bence Jones ou chaînes légères libres Mc (kappa ou lambda) dans l'urine ou le sérum humains par électrophorèse capillaire. Dans ce mode de réalisation, lorsque ces antisérums sont mis en oeuvre, l'échantillon est testé sur 6 pistes : une piste de référence (contenant seulement le milieu des anticorps, sans les anticorps), 5 pistes permettant de caractériser la ou les bandes Mc : présence de chaînes lourdes à l'aide de l'antisérum Trivalent, et type de chaîne légère (kappa ou lambda ; libre ou liée).

Les mélanges d'anticorps modifiés peuvent être obtenus par modification d'un mélange d'anticorps brut, ou par mélange d'anticorps préalablement modifiés par type d'anticorps. Dans le cadre de l'invention, la réalisation d'un mélange d'anticorps préalablement modifiés pour être surchargés négativement est préférée.

Dans le cadre de la réalisation de l'invention, la présentation des résultats de la séparation électrophorétique peut être faite comme illustré dans les exemples, sous la forme de profils électrophorétiques dont le pic correspondant à la protéine identifiée par une réaction immunologique spécifique avec les anticorps surchargés négativement a disparu du profil.

Alternativement, ces résultats peuvent être présentés sous la forme de profils électrophorétiques ne faisant apparaître que le pic correspondant à la protéine identifiée par une réaction immunologique spécifique avec les anticorps surchargés négativement. Dans le cadre de la mise en oeuvre du procédé de l'invention, ce pic disparait du profil des protéines séparées. Les résultats sont cependant traités pour ne faire apparaître que ce pic.

Pour ce faire, par des techniques appropriées de traitement de données, on soustrait chaque profil électrophorétique d'une partie aliquote de l'échantillon considéré traitée par un anticorps modifié surchargé négativement, du profil d'une partie aliquote non traitée, du même échantillon. La soustraction de tous les profils des parties aliquotes traitées conduit à une image de type de celles que l'on obtient dans une immunofixation.

### EXEMPLES ET FIGURES

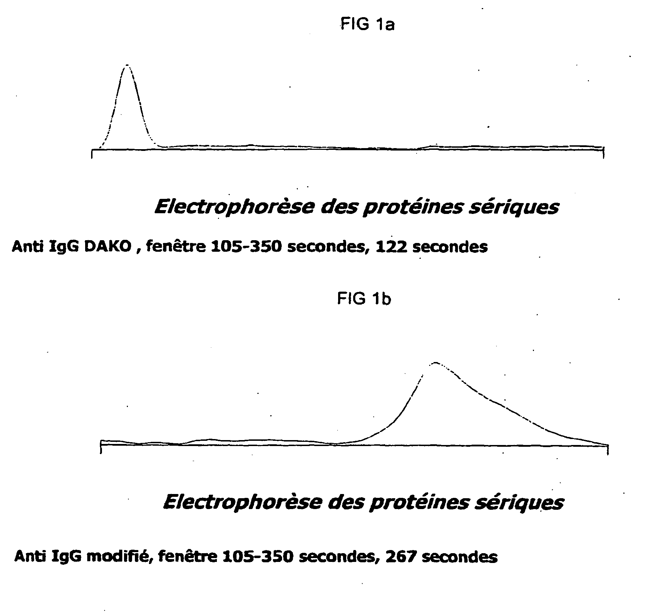

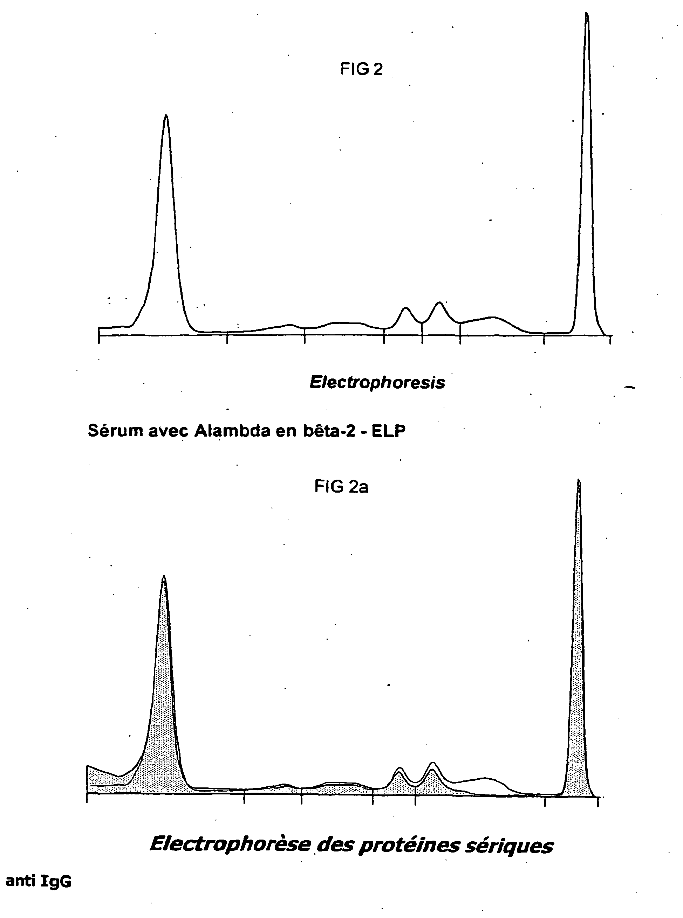

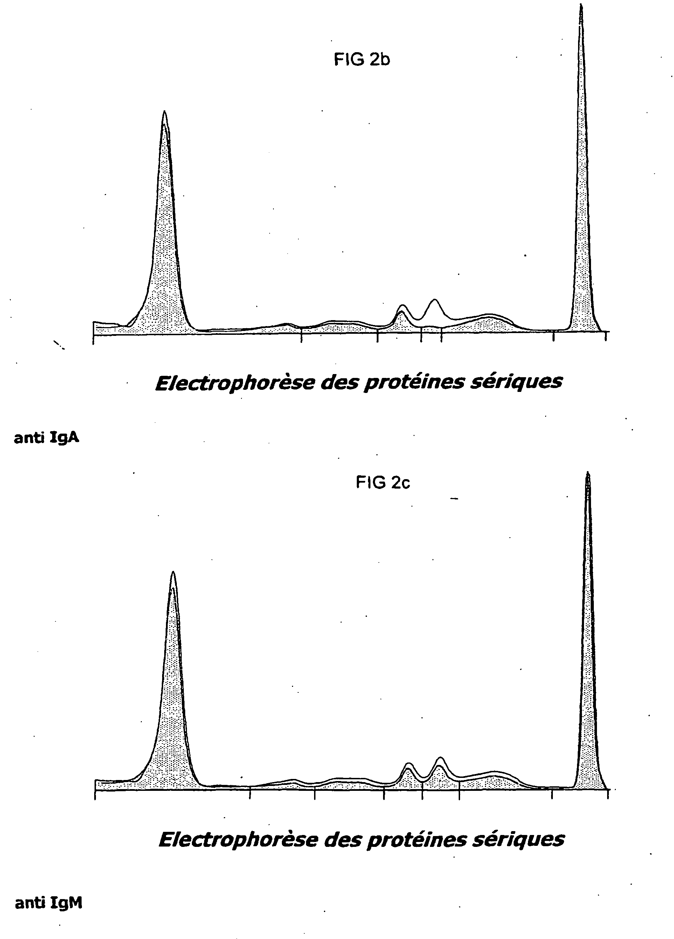

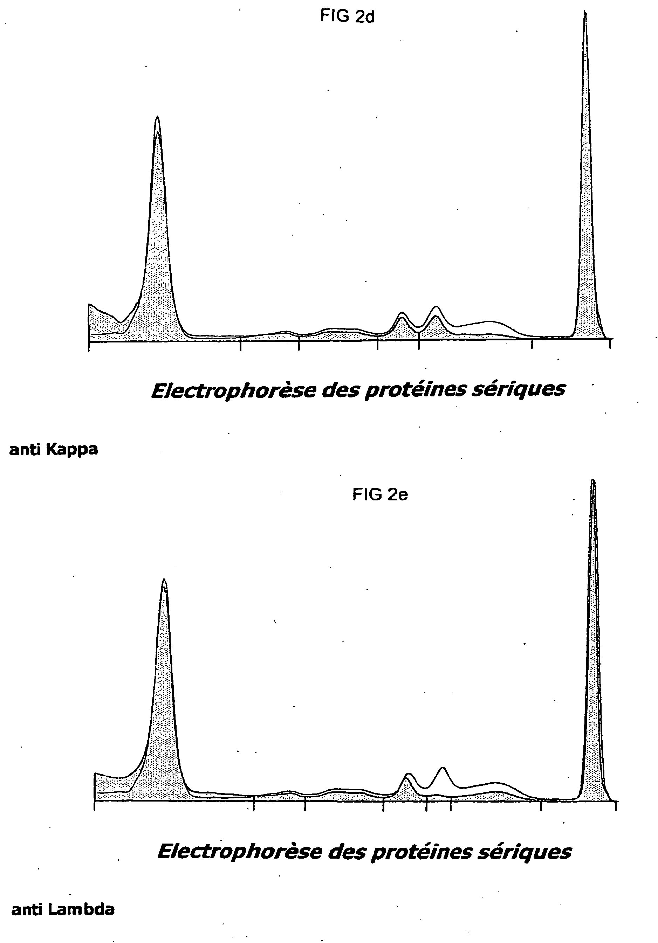

Les figures 1 à 13 illustrent des exemples de détection de protéines monoclonales dans différents échantillons biologiques et le typage de ces protéines monoclonales, et améliorent de ce fait le pronostic et le traitement de ces pathologies. Sur les figures 2 à 13, la surface grisée du profil représente les protéines sériques séparées en présence des anticorps de l'invention ; le profil des protéines sériques séparées hors de la présence des anticorps apparaît également selon un tracé plus fin. Le profil des protéines séparées non traitées avec les anticorps de l'invention apparaît également seul dans chaque figure, sous la désignation ELP.

### ELECTROPHORESE CAPILLAIRE

L'électrophorèse capillaire d'échantillons cliniques est réalisée sur un appareil d'EC équipé de 8 capillaires en silice fondue de diamètre interne 25 microns (CAPILLARYS, Sebia). La détection est réalisée à 200 nm. Les échantillons sont placés dans le passeur d'échantillons de l'appareil et injectés automatiquement par injection hydrodynamique. La séparation des échantillons est réalisée en moins de 5 minutes en appliquant un champ électrique d'environ 400 V/cm. Le capillaire est lavé avant chaque analyse par la soude 0,25 M, puis par le tampon d'analyse.

### Protocole pour la modification des anticorps par l'anhydride tricarboxylique

a) 10 mL de l'antisérum à greffer (anti-human IgG, A, M, anti Kappa ou Lambda DAKO Danemark à 10 g/l ont été dialysés contre une solution tampon phosphate 100mM pH 7,4.
b) une solution d'anhydride de 1,2,4-benzènetricarboxylique à 100 mM dans le DMF a été préparée.
c) La solution dialysée d'anticorps a alors été mélangée à de la soude 5N puis la solution d'anhydride dans le DMF (213 µl de soude 5N + 10 ml de solution d'anticorps + 5,33 ml d'anhydride dans le DMF).
d) Le milieu réactionnel a alors été placé en agitation douce sur un balancier pendant une heure à 37°C.
e) Le mélange a alors été dialysé contre la solution tampon de phosphate 100mM pH 7,4 avec plusieurs changements du bain de dialyse.
f) Une fois la dialyse terminée, le boudin a été placé dans du PEG 6000 en poudre pendant 1 h.
g) Une fois la concentration terminée, ce dernier a alors été dialysé contre du phosphate 100mM pH 7,4 + 1 g/L d'azoture de sodium.
h) Le volume du boudin a été ramené à 10mL par une solution de phosphate 100mM pH 7,4 + 1g/L d'azoture de sodium.
i) La solution obtenue a été filtrée sur une membrane de porosité 0,45 µm puis est conservée à 4°C . Tout autre milieu de conservation peut être utilisé à condition que sa force ionique soit suffisante pour assurer la conservation des anticorps (c'est à dire proche de celle de l'eau physiologique).

### Protocole pour la modification des anticorps par l'acide mellitique

a) 10 mL de l'antisérum à greffer (anti-human IgG, A, M, anti Kappa ou Lambda DAKO Danemark à 10 g/l ont été dialysés contre une solution tampon PBS 40 mM pH 7,4.
b) Ajouter 50 µl/ml de soude 5N ;
c) Ajouter (18 mg/ml d'acide mellitique puis 50 mg/ml d'EDCI) ;
d) Le milieu réactionnel a alors été placé en agitation douce sur un balancier pendant une heure à 37°C.
e) Le mélange a alors été dialysé contre la solution tampon de phosphate 100mM pH 7,4 avec plusieurs changements du bain de dialyse.
f) Une fois la dialyse terminée, le boudin a été placé dans du PEG 6000 en poudre pendant 1h.
g) Une fois la concentration terminée, ce dernier a alors été dialysé contre du phosphate 100mM pH 7,4 + 1 g/L d'azoture de sodium.
h) Le volume du boudin a été ramené à 10mL par une solution de phosphate 100mM pH 7,4 + 1g/L d'azoture de sodium.
i) La solution obtenue a été filtrée sur une membrane de porosité 0,45 µm et le tout conservé à 4°C.

### Protocole pour l'immunotypage d'un sérum

Le Capillarys (SEBIA) est un appareil qui effectue 8 analyses capillaires en parallèle. La barrette immunotypage est une barrette CAPILLARYS spécifique au typage constituée de 6 puits de 100 µl et d'un double puits de 400 µl. Elle contient 60 µL de phosphate 100 mM dans le puits 1, 60µL d'anti-IgG modifié dans le puits 2, d'anti-IgA modifié dans le puits 3, d'anti-IgM modifié dans le puits 4, d'anti-Kappa modifié dans le puits 5, d'anti-Lambda modifié dans le puits 6, le double puits 7/8 contenant 380 µL de diluant immunotypage (alcool benzylique 0.02M dans le tampon d'analyse Sebia utilisé.
a) Le tube de sérum à typer a été mis en position 1 sur le portoir CAPILLARYS sur lequel a été placée une barrette « immunotypage ».
b) Le portoir a été introduit dans l'appareil : l'automate a alors réalisé une dilution 1/20 du sérum dans le double puits 7/8, le double puits a été homogénéisé et 40 µl ont été répartis dans chacun des puits 1 à 6 en homogénéisant chacun de ces puits.
c) L'analyse capillaire a ensuite été effectuée dans les conditions suivantes :
   Le tampon d'analyse peut être le tampon Sebia Capillarys B1B2+ ou le tampon Sebia Capillarys Protein :
   Tampon B1B2+ : Température 32°C, tension de migration 7 kV, fenêtre d'acquisition 100-235s, injection 250 mbars * 5s ;
   Tampon Sebia Capillarys Protein (e) : Température 37°C, tension de migration 6,5 kV, fenêtre d'acquisition 110-275s, injection 250 mbars * 5s ;
d) L'analyse capillaire obtenue dans le puits 1 a été comparée à celle des cinq puits suivants afin de typer le sérum analysé.

### Aménagement des conditions opératoires pour des sérums particuliers:

Si le sérum possède une fraction gamma faible, la dilution dans le diluant en b) est au 1/10^{ème}. La fraction gamma sera considérée comme faible si son analyse sur Capillarys en tampon B1B2+ donne une valeur inférieure aux normales définies pour cette fraction avec ce tampon, soit environ inférieure à 10% (ou < 7,5 g/l).

Si le sérum possède une faction gamma supérieure à environ 20 g/L, la dilution dans le diluant en b) est au 1140^{ème}.

### Variante au protocole pour l'immunotypage d'un sérum : immunosoustraction in-capillaire

Dans ce cas, 2 barrettes sont utilisées :
1 barrette constituée de 6 puits contenant : 100 µl de phosphate 100 mM dans le puits 1, 100 µl d'anti-IgG modifié dans le puits 2, d'anti-IgA modifié dans le puits 3, d'anti-IgM modifié dans le puits 4, d'anti-kappa modifié dans le puits 5, d'anti-lambda modifié dans le puits 6 ;
1 barrette pour la dilution du sérum constituée de 7 puits contenant : 380 µl de diluant immunotypage (alcool benzylique 0,02 M dans le tampon Sebia Capillarys B1B2+) dans le double puits 7/8 ;
   a) Un premier portoir contenant la barrette d'antisérums a été introduit dans l'appareil : l'automate a alors effectué une injection de ces anticorps dans les capillaires (250 mbars *5s).
   b) Un deuxième portoir contenant la barrette pour la dilution de sérum et un tube de sérum en position 1 a ensuite été introduit dans l'appareil : l'automate a alors réalisé une dilution 1/20^{ème} dans le double puits 7/8, le double puits a été homogénéisé et réparti dans les puits 1 à 6 (40 µl de sérum au 1/20^{ème} + 60 µl de tampon d'analyse par puits) ; les sérums dilués ont alors été injectés dans les capillaires (250 mbars * 5s).
   c) Le tampon d'analyse peut être le tampon Sebia Capillarys B1B2+ ou le tampon Sebia Capillarys Protein(e) :
      Tampon B1B2+: Température 32°C, tension de migration 7kV, fenêtre d'acquisition 100-235s ;
      Tampon Sebia Capillarys Protein(e) : Température 37°C, tension de migration 6.5 kV, fenêtre d'acquisition 110-275s;
   d) l'analyse capillaire obtenue dans le puits 1 a été comparée à celle des cinq puits suivants afin de typer le sérum.

### Résultats :

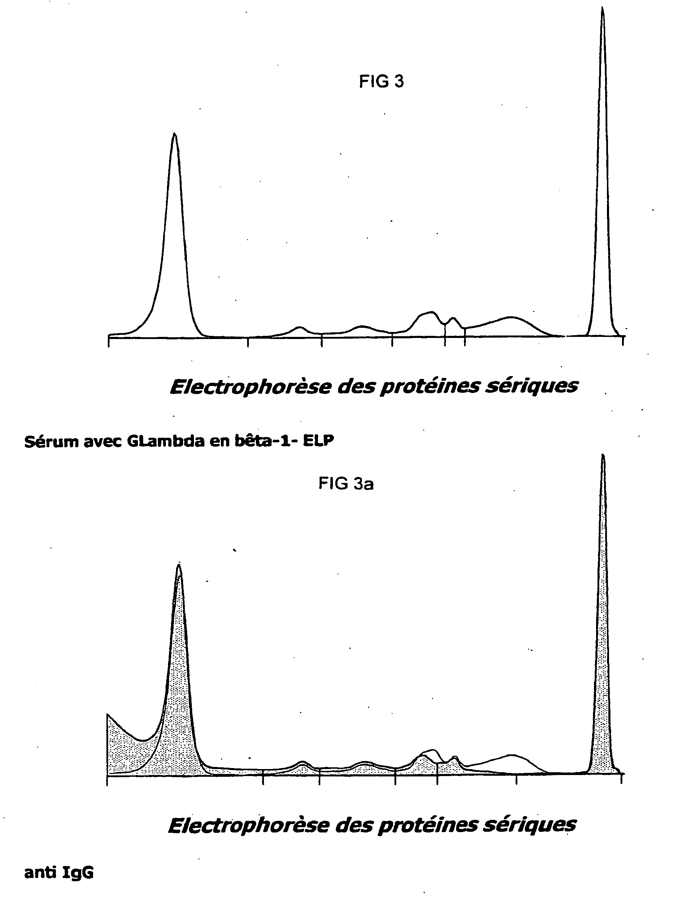

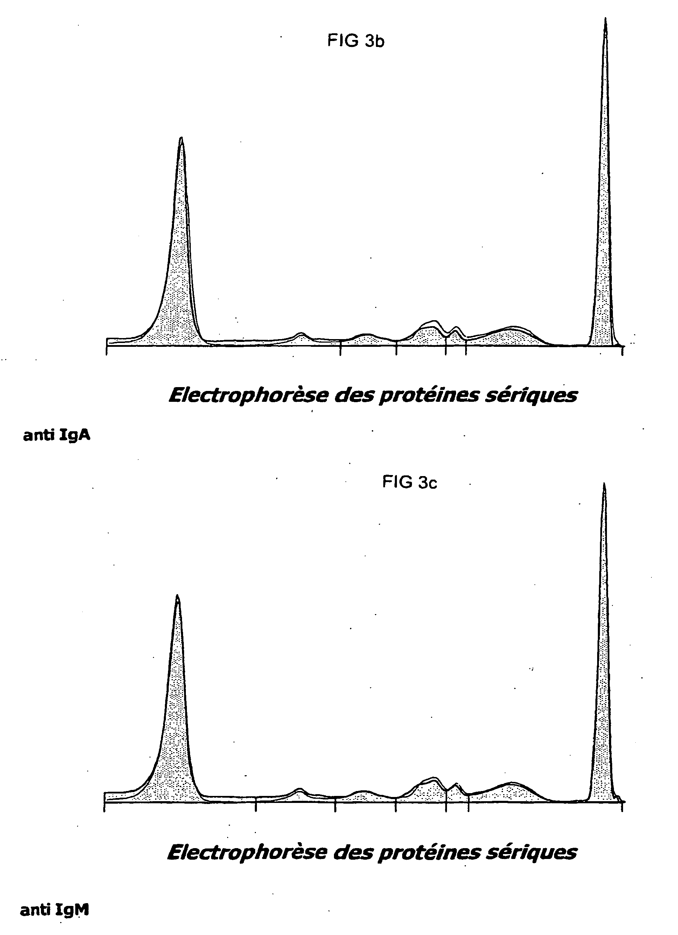

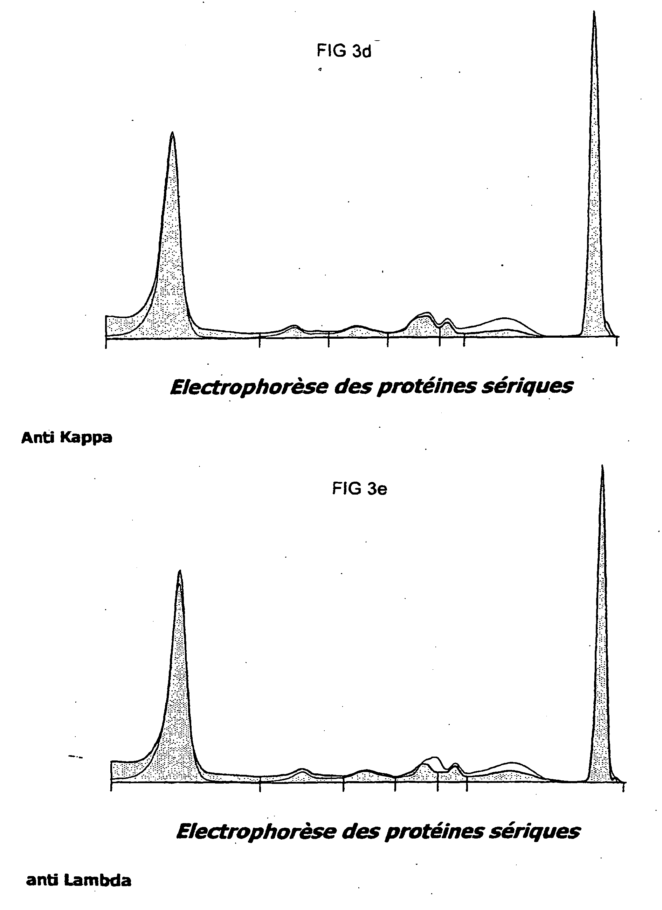

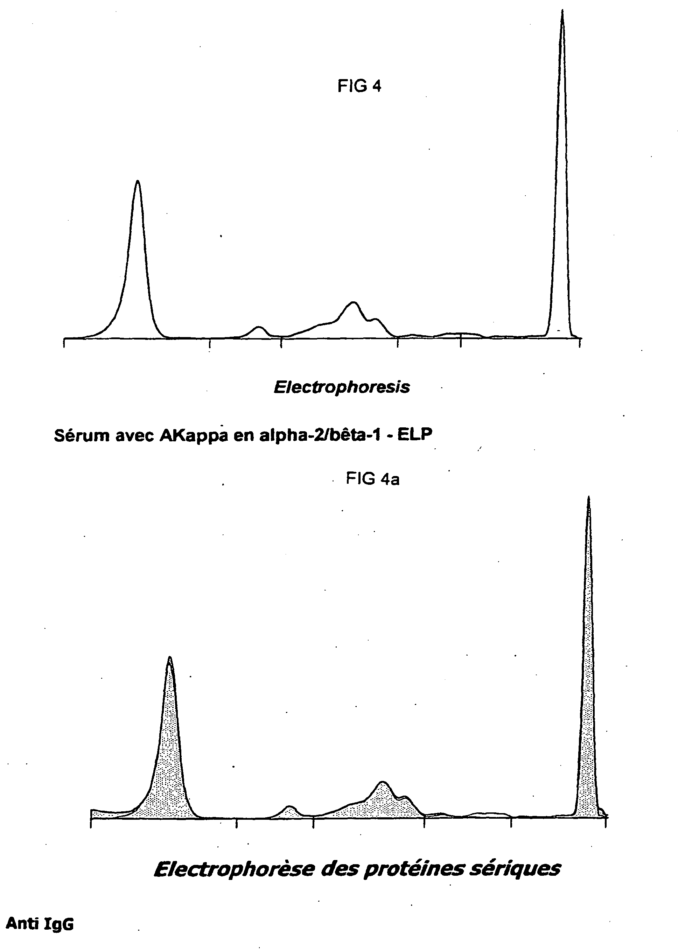

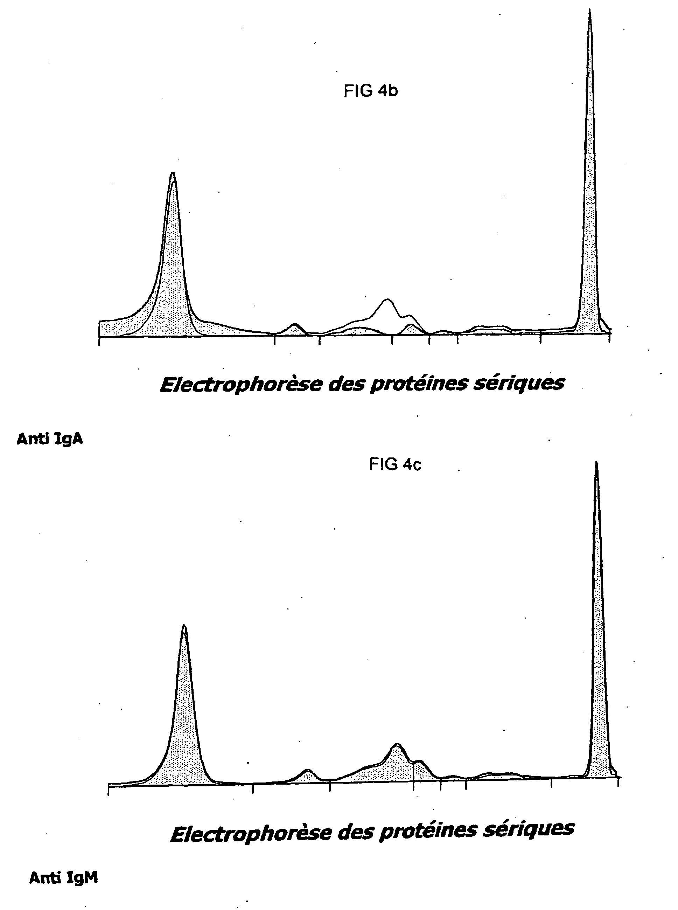

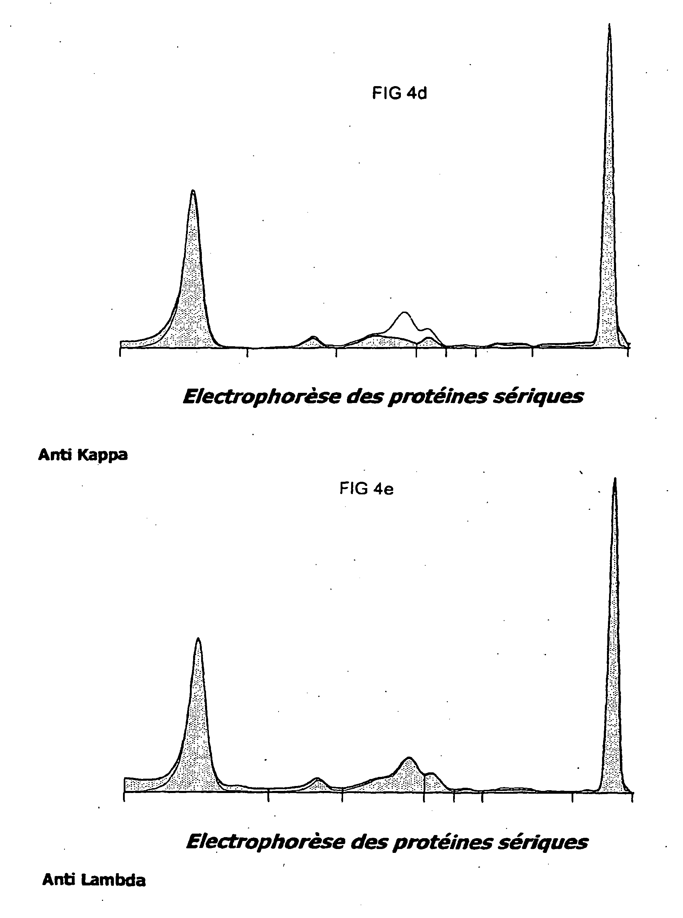

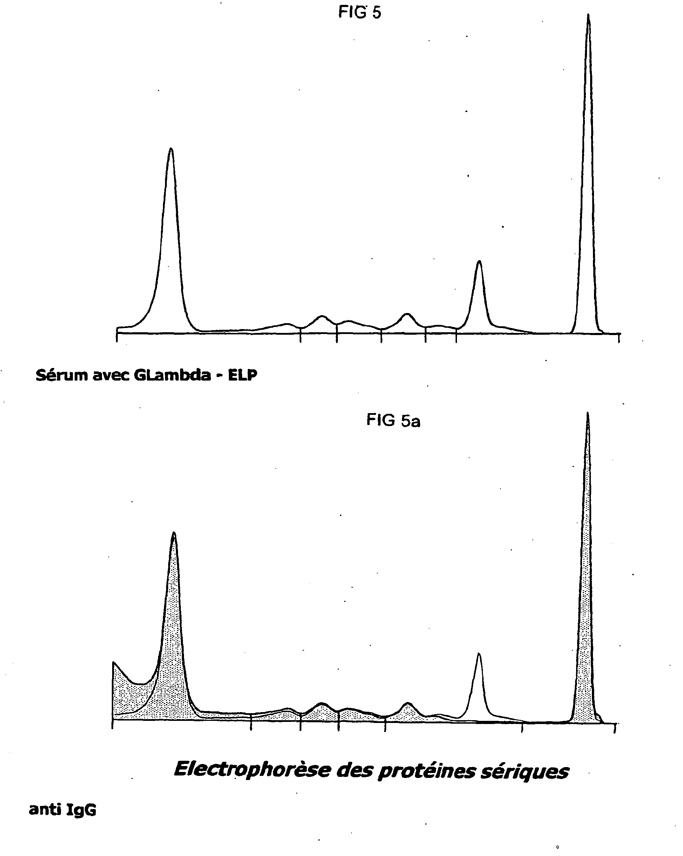

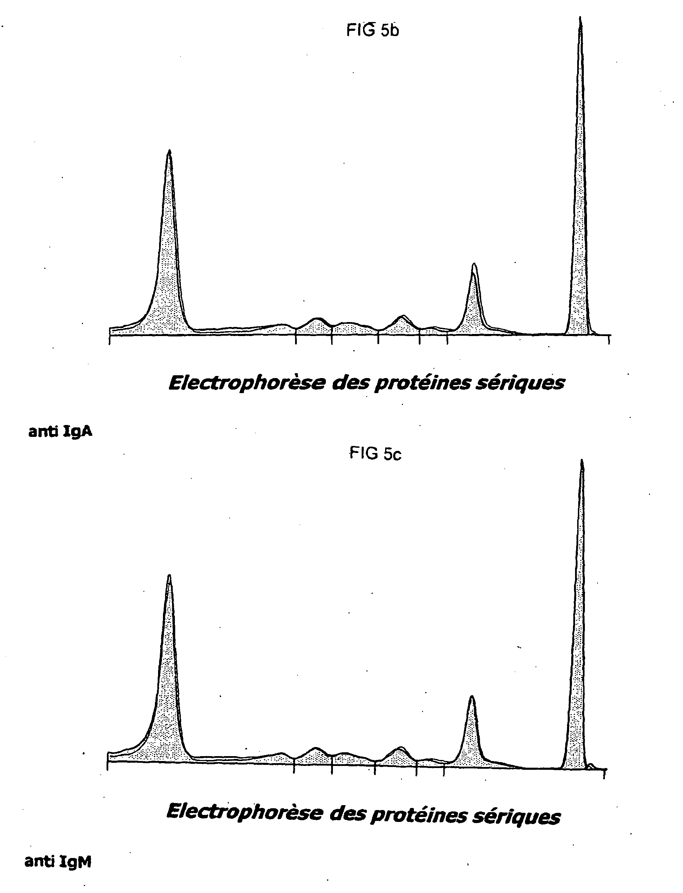

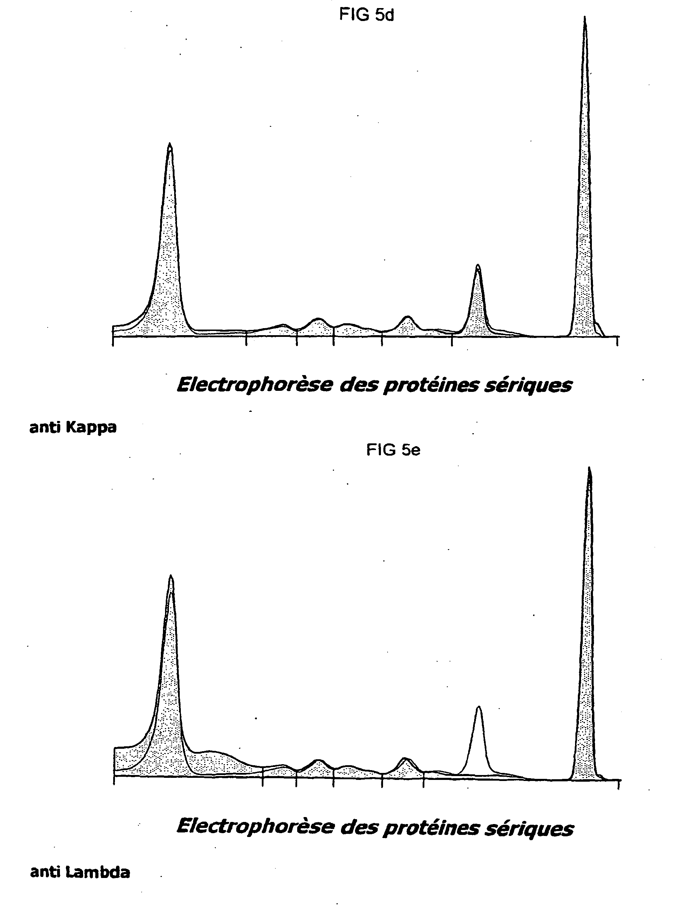

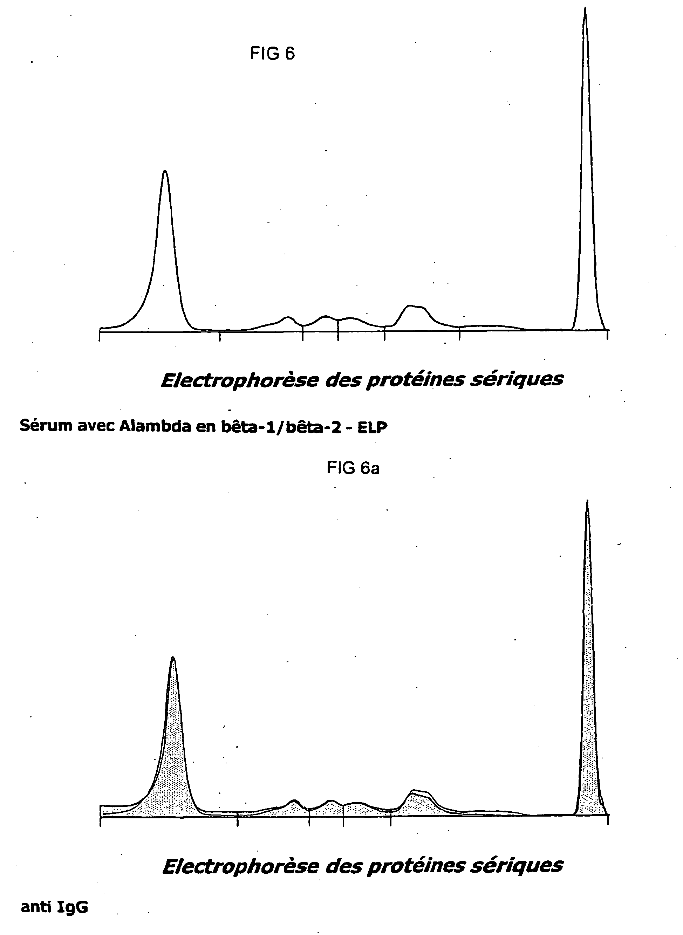

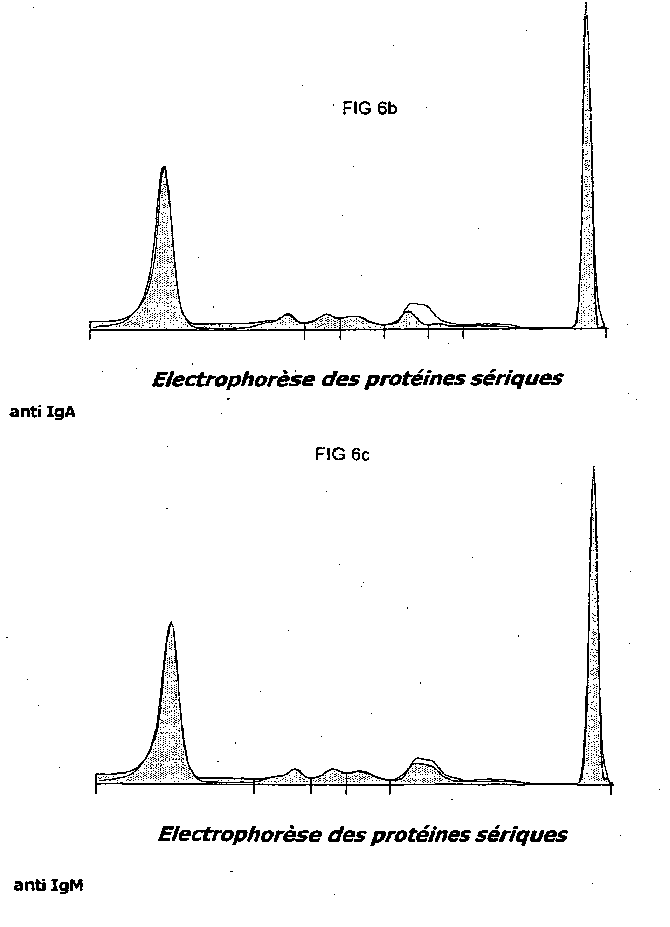

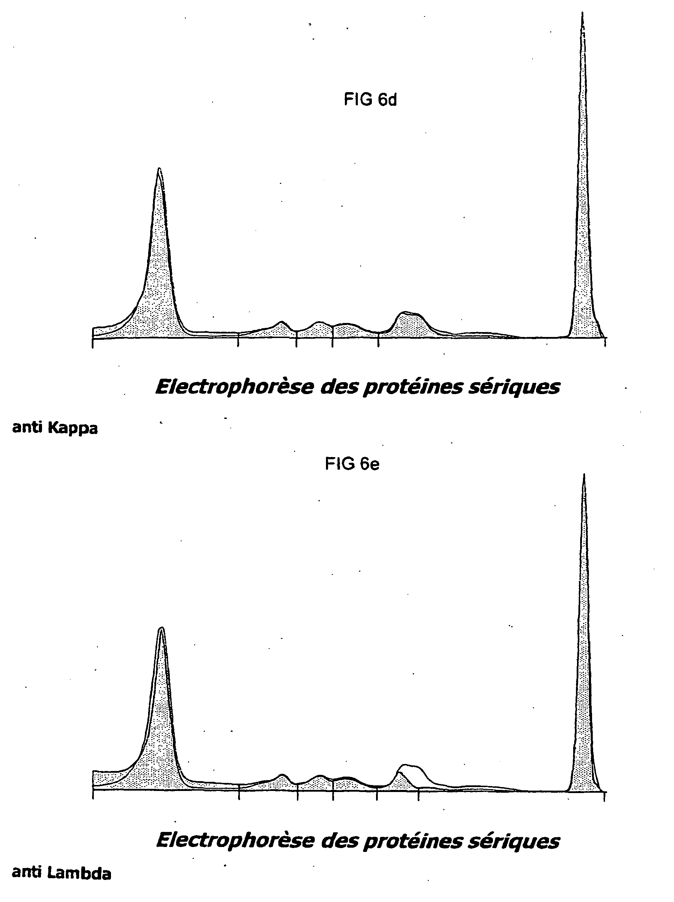

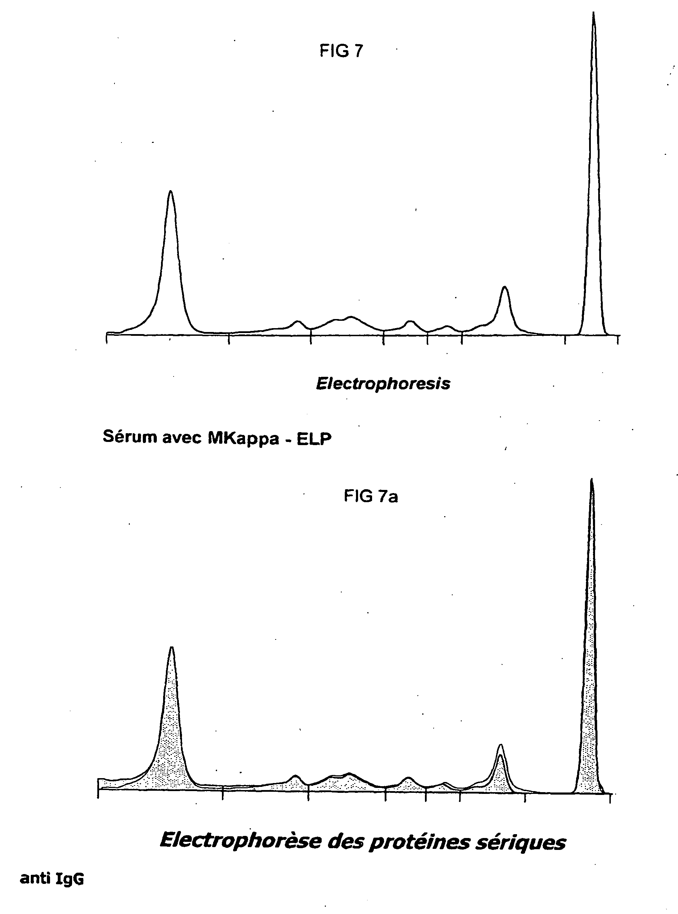

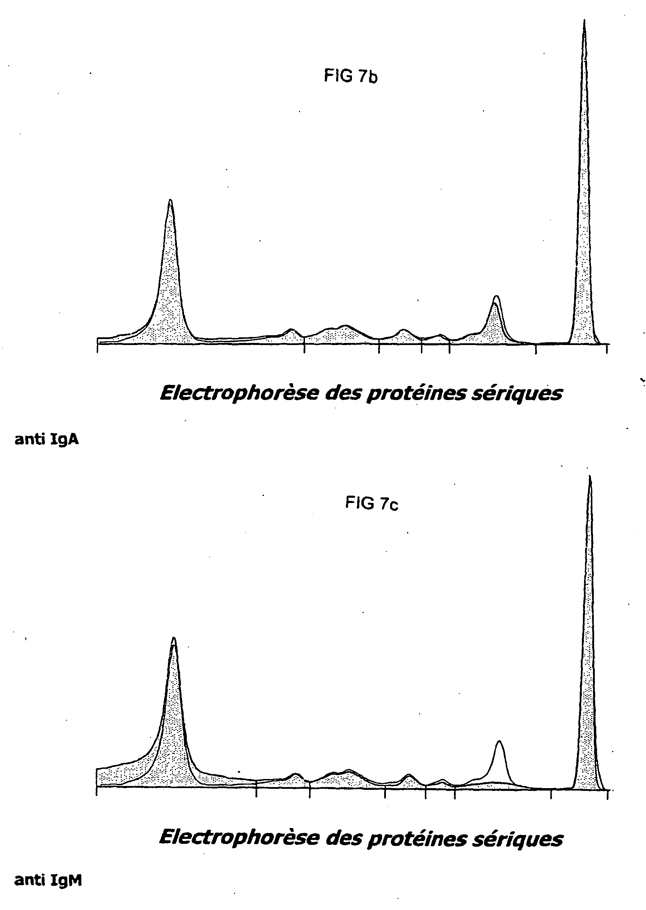

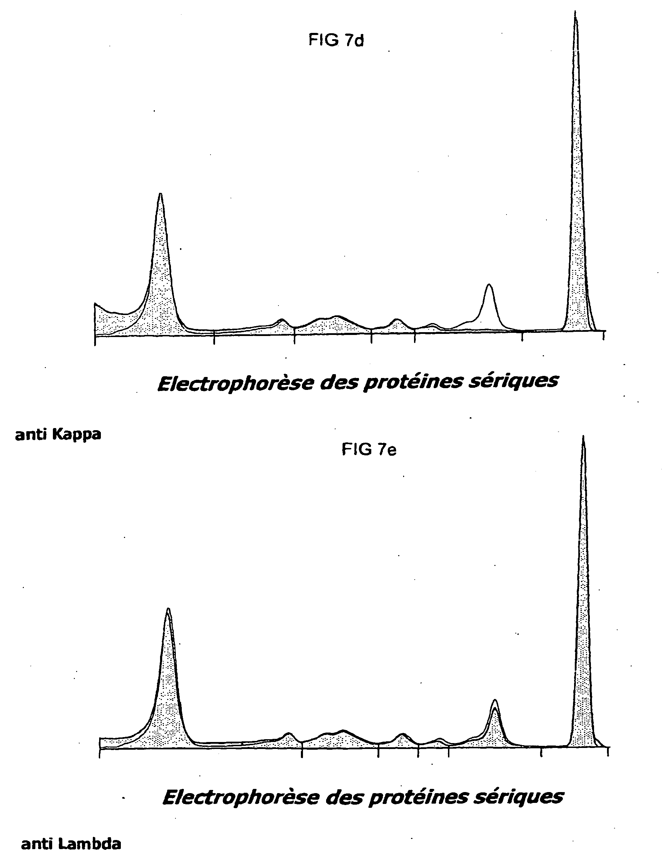

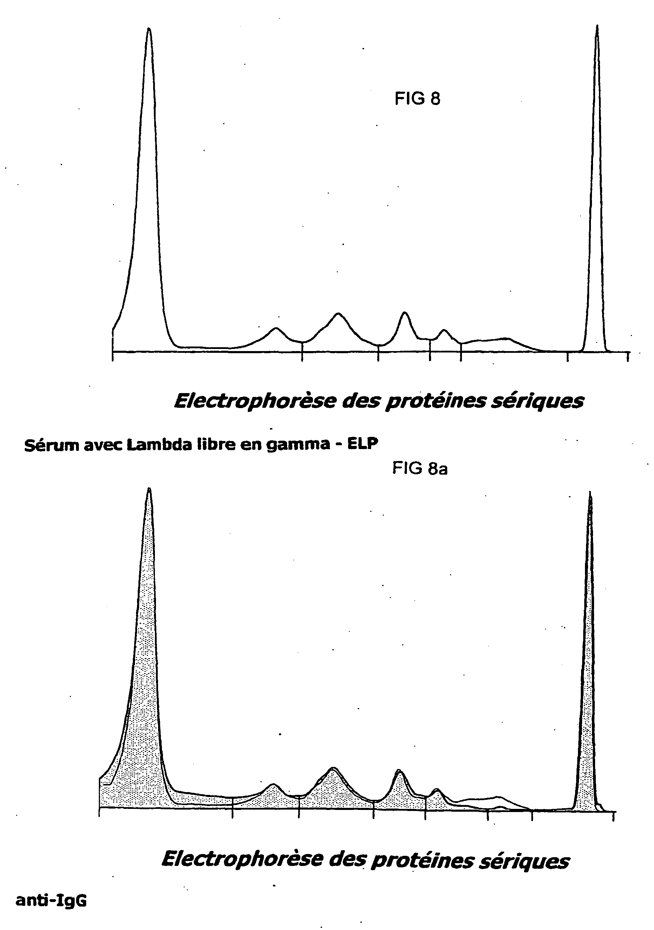

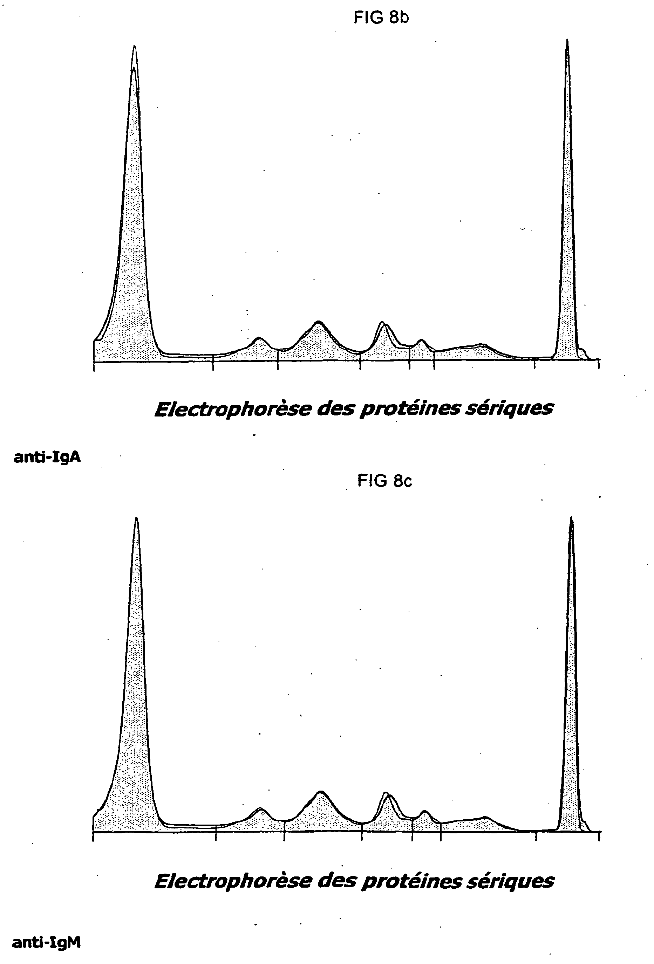

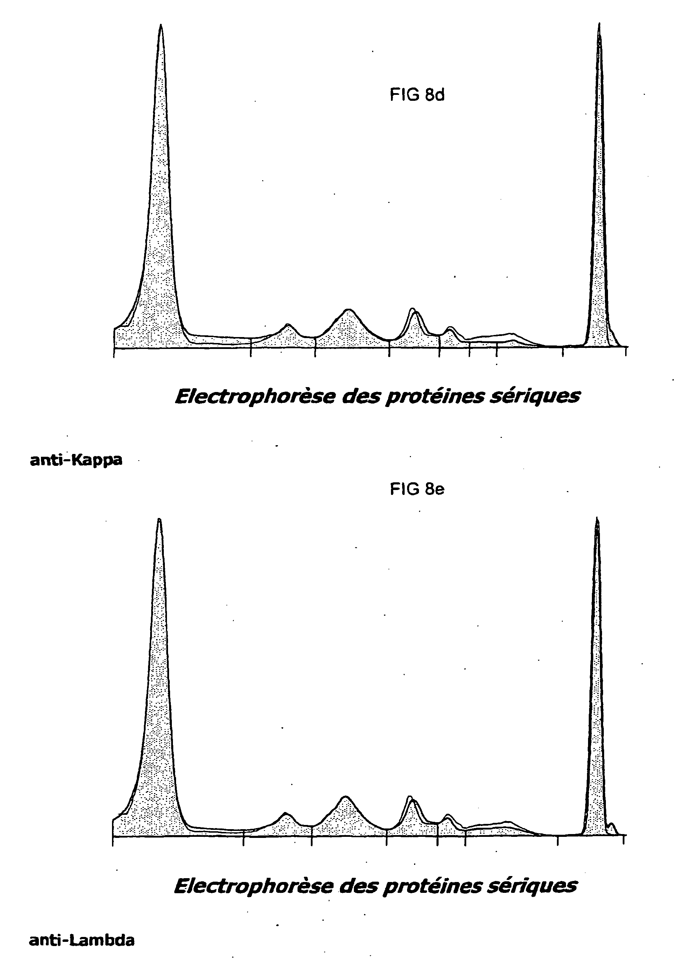

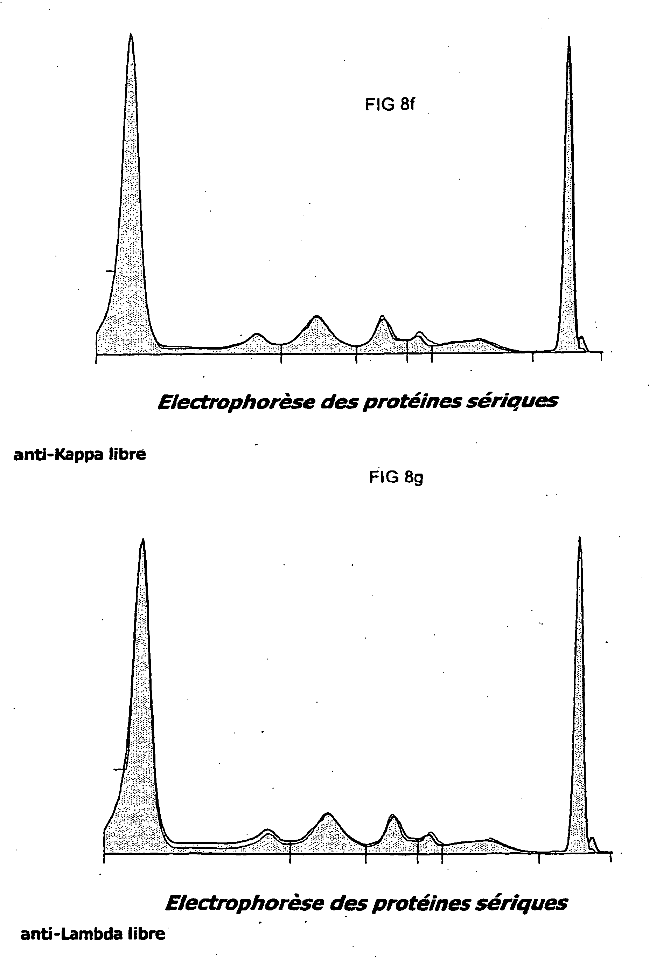

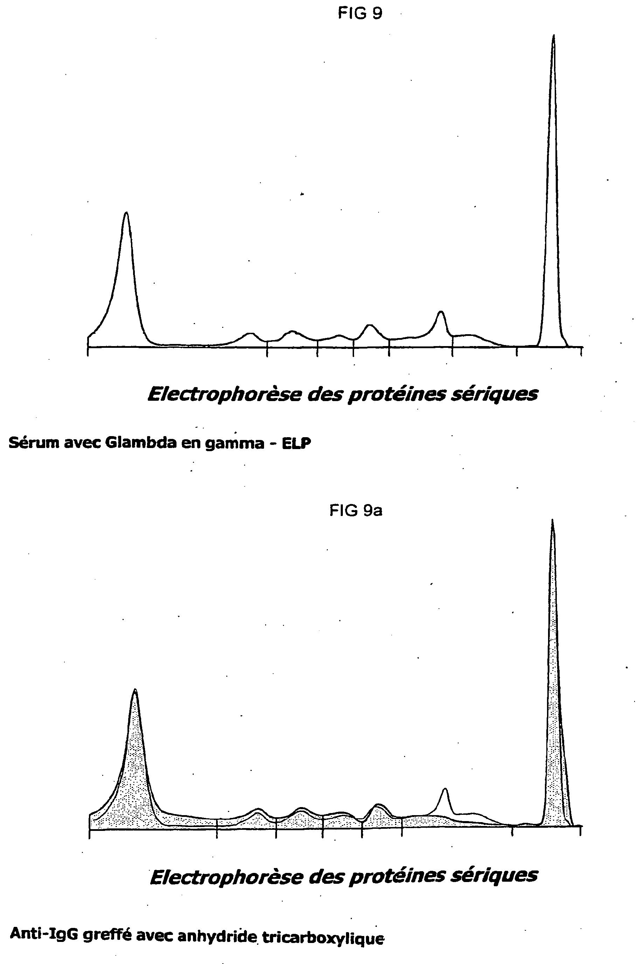

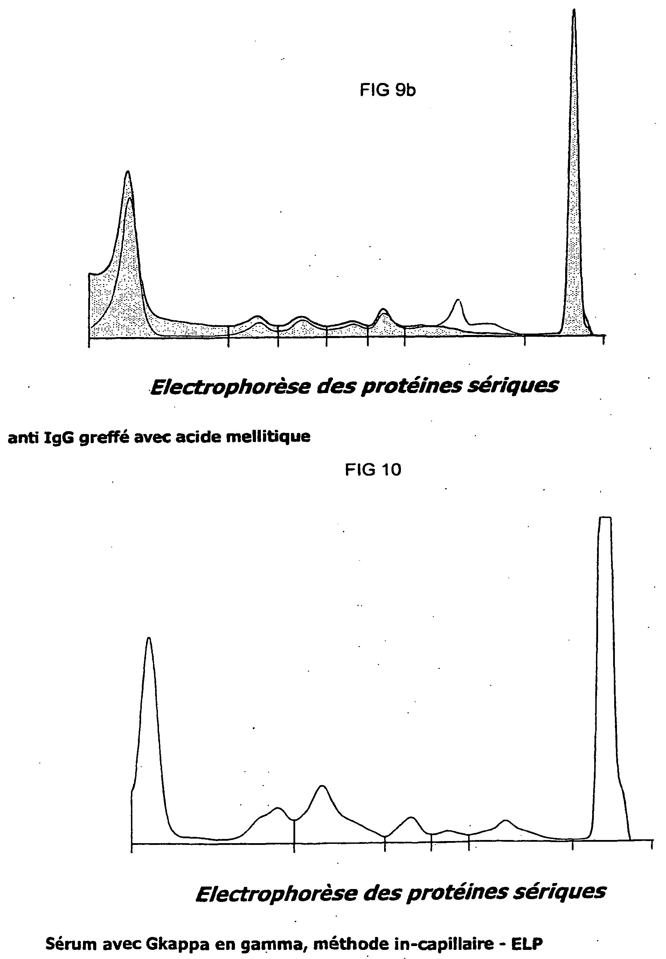

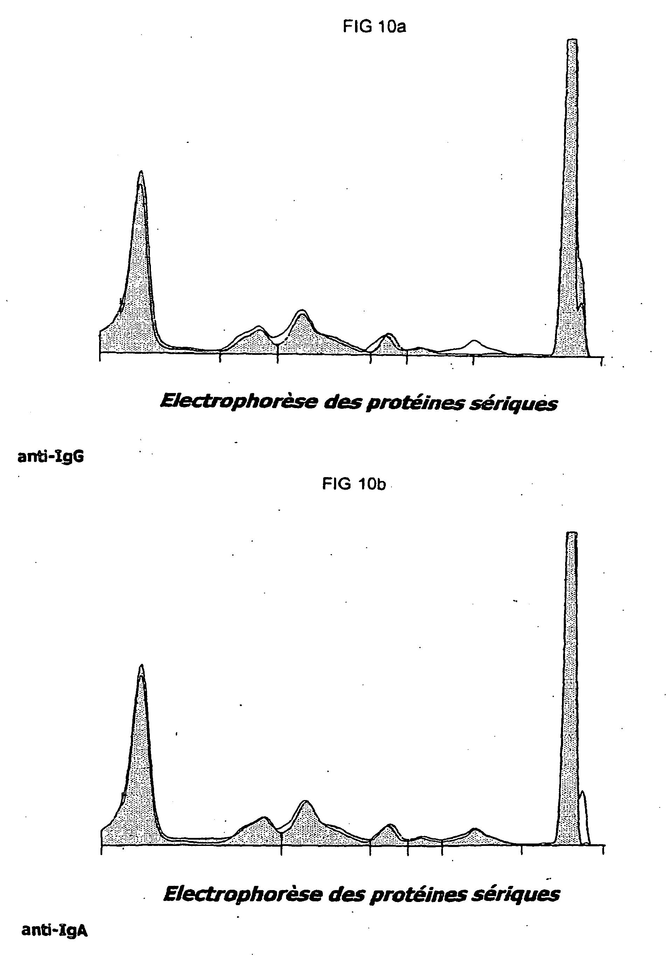

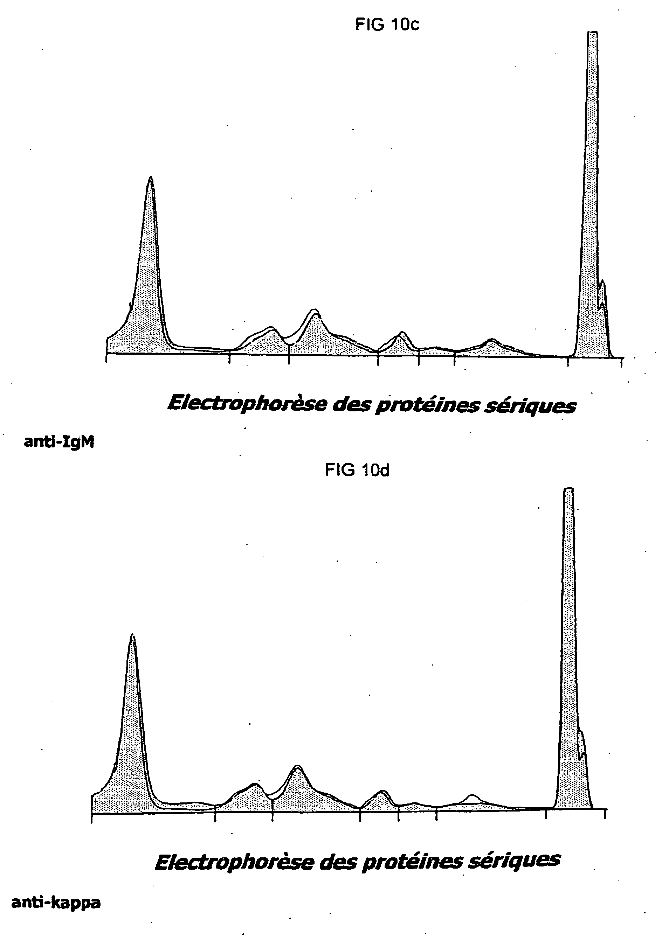

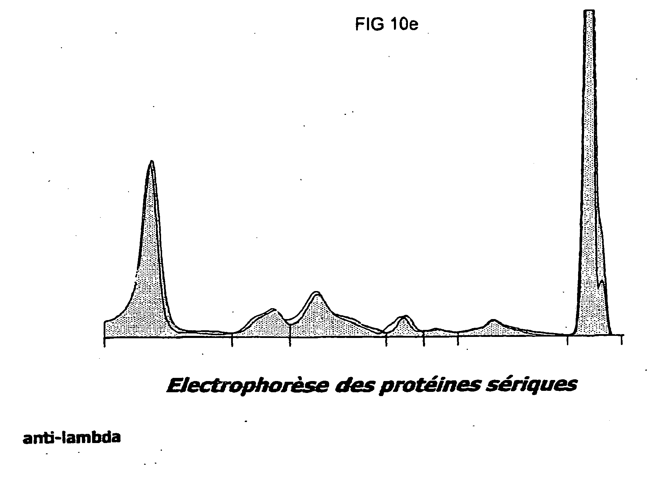

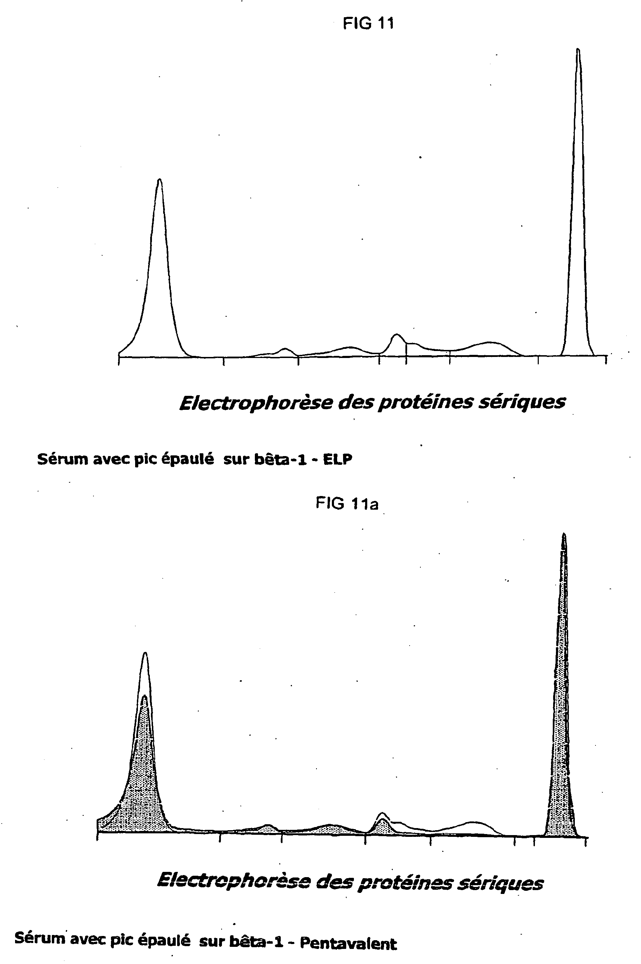

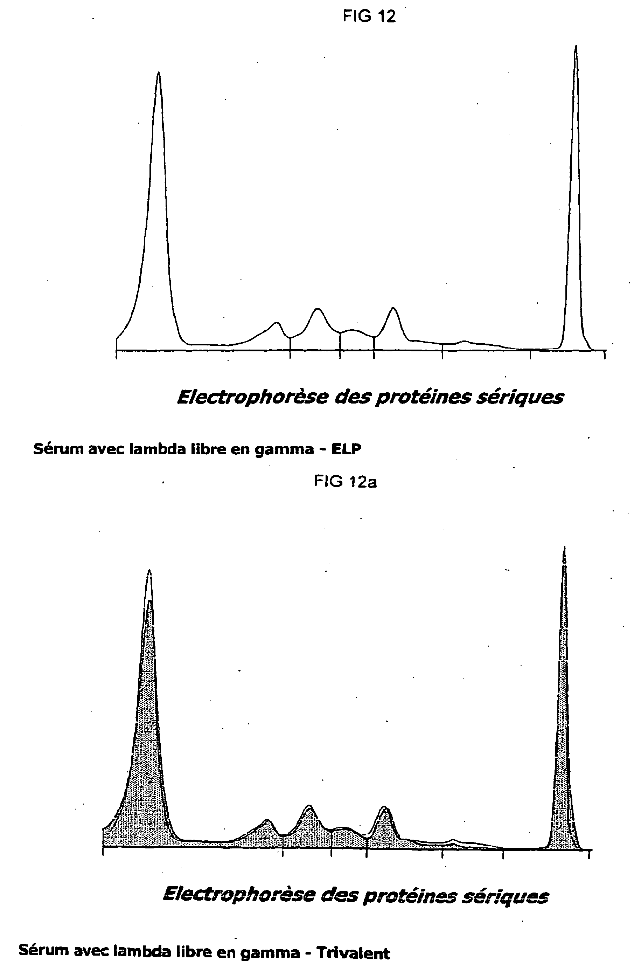

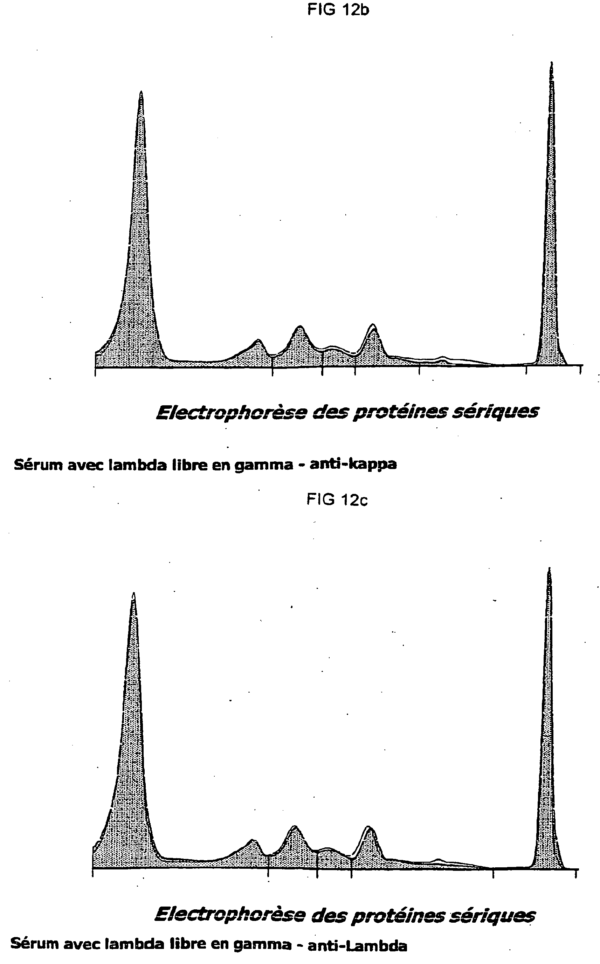

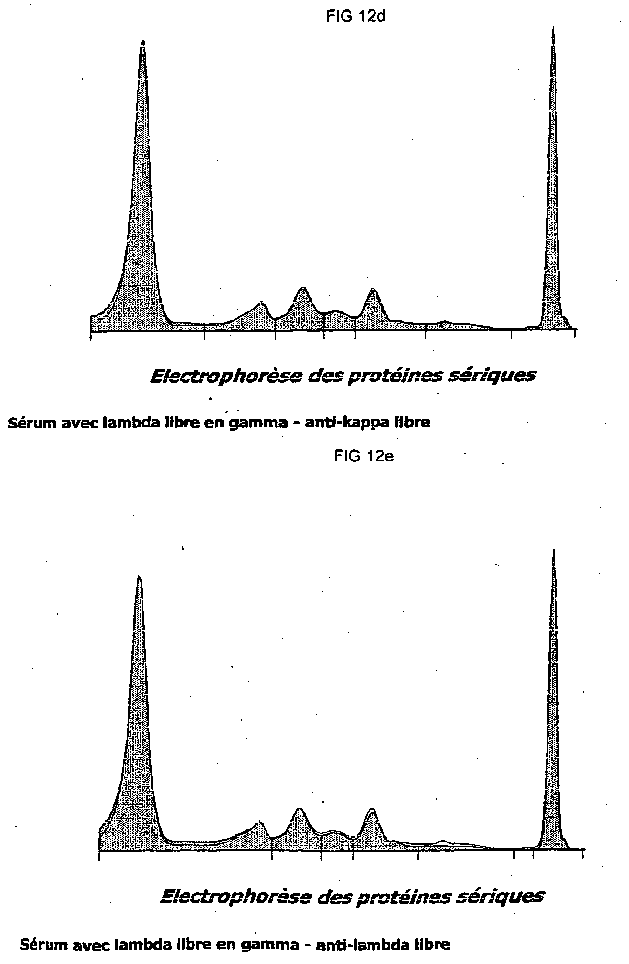

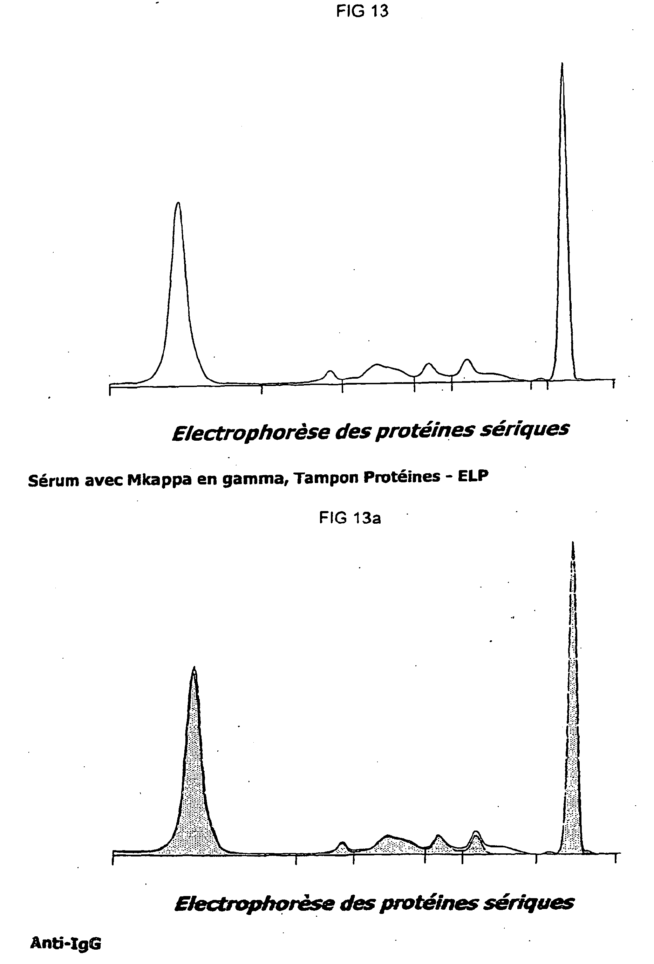

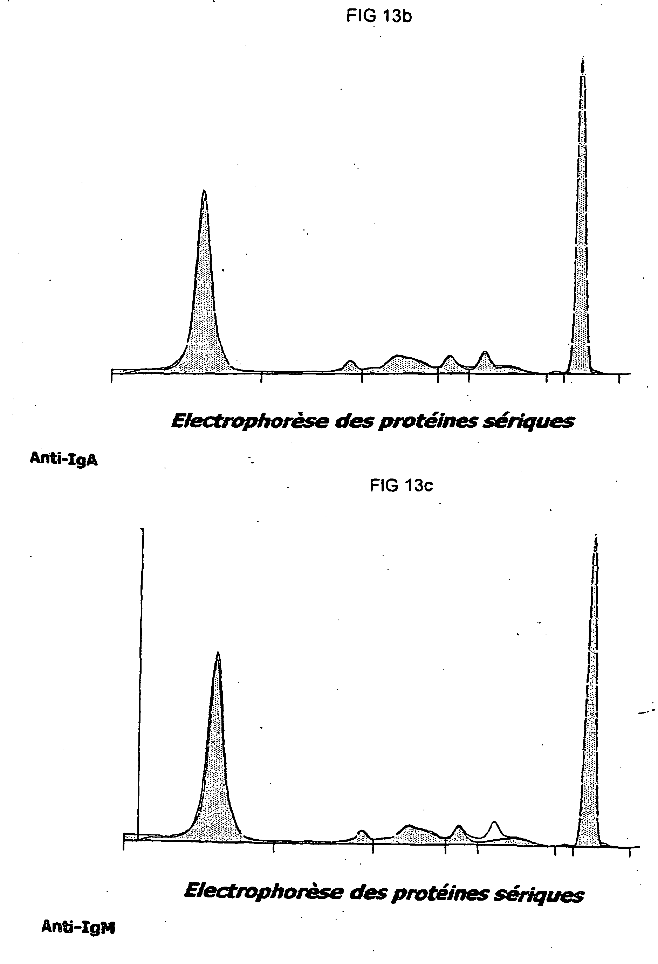

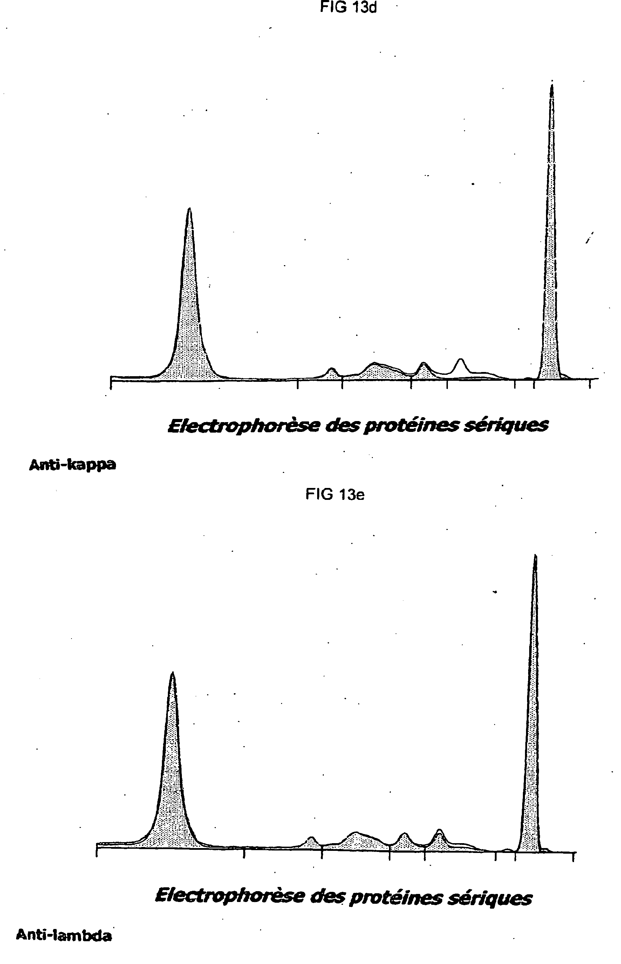

o Exemple 1 : analyse d'un anti-human IgG DAKO avant et après modification par l'anhydride 1,2,4-benzènetricarboxylique sur Capillarys avec le tampon Sebia Capillarys B1B2+ (fenêtre d'acquisition 105-350s) ; la modification de l'anticorps permet de fortement retarder sa migration : son temps de migration passe de 122 à 267 s (Figures 1 a et 1b).
o Exemple 2 : immunotypage d'un sérum contenant une protéine monoclonale de type Alambda migrant en bêta-2 sur Capillarys avec le tampon Sebia Capillarys B1B2+ (fenêtre d'acquisition 100-235s): 6 aliquotes de ce sérum ont été mélangées avec 5 anticorps modifiés par l'anhydride 1,2,4-benzènetricarboxylique (anti-IgG, -IgA, -IgM, -kappa, - lambda) et avec le milieu de ces anticorps (sans anticorps ; piste ELP). Par comparaison avec la piste ELP (en noir ; Figure 2), on observe clairement la disparition du pic monoclonal en bêta-2 pour les aliquotes traitées par les anti-IgA et anti-lambda. Sur les autres pistes, seule la diminution du fond polyclonal est observée. On notera également l'absence d'interférence des immuncomplexes et des anticorps modifiés avec la migration des globulines. Le pic migrant avant les gammas est un marqueur d'injection facilitant la superposition des profils.
o Exemple 3 : immunotypage d'un sérum contenant une protéine monoclonale de type G lambda migrant en bêta-1 sur Capillarys avec le tampon Sebia Capillarys B1B2+ (fenêtre d'acquisition 100-235s) : 6 aliquotes de ce sérum ont été mélangées avec 5 anticorps modifiés par l'anhydride 1,2,4-benzènetricarboxylique (anti-IgG, -IgA, -IgM, -kappa, - lambda) et avec le milieu de ces anticorps (sans anticorps ; piste ELP). Par comparaison avec la piste ELP (en noir ; Figure 3), on observe clairement la disparition du pic monoclonal en bêta-1 pour les aliquotes traitées par les anti-IgG et anti-lambda. Sur les autres pistes, seule la diminution du fond polyclonal est observée. On notera également l'absence d'interférence des immuncomplexes et des anticorps modifiés avec la migration des globulines. Le pic migrant avant les gammas est un marqueur d'injection facilitant la superposition des profils.
o Exemple 4: immunotypage d'un sérum contenant une protéine monoclonale de type A kappa migrant entre bêta-1 et alpha-2 sur Capillarys avec le tampon Sebia Capillarys B1B2+ (fenêtre d'acquisition 100-235s) : 6 aliquotes de ce sérum ont été mélangées avec 5 anticorps modifiés par l'anhydride 1,2,4-benzènetricarboxylique (anti-IgG, -IgA, - IgM, -kappa, -lambda) et avec le milieu de ces anticorps (sans anticorps ; piste ELP). Par comparaison avec la piste ELP (en noir ; Figure 4), on observe clairement la disparition du pic monoclonal en bêta-1/alpha-2 pour les aliquotes traitées par les anti-IgA et anti-kappa. Sur les autres pistes, seule la diminution du fond polyclonal est observée. On notera également l'absence d'interférence des immuncomplexes et des anticorps modifiés avec la migration des globulines. Le pic migrant avant les gammas est un marqueur d'injection facilitant la superposition des profils.
o Exemple 5 : immunotypage d'un sérum contenant une protéine monoclonale de type G lambda migrant en gamma sur Capillarys avec le tampon Sebia Capillarys B1B2+ (fenêtre d'acquisition 100-235s) : 6 aliquotes de ce sérum ont été mélangées avec 5 anticorps modifiés par l'anhydride 1,2,4-benzènetricarboxylique (anti-IgG, -IgA, -IgM, -kappa, - lambda) et avec le milieu de ces anticorps (sans anticorps ; piste ELP). Par comparaison avec la piste ELP (en noir ; Figure 5), on observe clairement la disparition du pic monoclonal en gamma pour les aliquotes traitées par les anti-IgG et anti-lambda. Sur les autres pistes, seule la diminution du fond polyclonal est observée. On notera également l'absence d'interférence des immuncomplexes et des anticorps modifiés avec la migration des globulines. Le pic migrant avant les gammas est un marqueur d'injection facilitant la superposition des profils.
o Exemple 6 : immunotypage d'un sérum contenant une protéine monoclonale de type A lambda migrant entre bêta-2 et bêta-1 sur Capillarys avec le tampon Sebia Capillarys B1B2+ (fenêtre d'acquisition 100-235s) : 6 aliquotes de ce sérum ont été mélangées avec 5 anticorps modifiés par l'anhydride 1,2,4-benzènetricarboxylique (anti-IgG, -IgA, - IgM, -kappa, -lambda) et avec le milieu de ces anticorps (sans anticorps ; piste ELP). Par comparaison avec la piste ELP (en noir ; Figure 6), on observe clairement la disparition du pic monoclonal en bêta-1/bêta-2 pour les aliquotes traitées par les anti-IgA et anti-lambda. Sur les autres pistes, seule la diminution du fond polyclonal est obervée. On notera également l'absence d'interférence des immuncomplexes et des anticorps modifiés avec la migration des globulines. Le pic migrant avant les gammas est un marqueur d'injection facilitant la superposition des profils.
o Exemple 7 : immunotypage d'un sérum contenant une protéine monoclonale de type M kappa migrant en gamma sur Capillarys avec le tampon Sebia Capillarys B1B2+ (fenêtre d'acquisition 100-235s) : 6 aliquotes de ce sérum ont été mélangées avec 5 anticorps modifiés par l'anhydride 1,2,4-benzènetricarboxylique (anti-IgG, -IgA, -IgM, -kappa, - lambda) et avec le milieu de ces anticorps (sans anticorps ; piste ELP). Par comparaison avec la piste ELP (en noir ; Figure 7), on observe clairement la disparition du pic monoclonal en gamma pour les aliquotes traitées par les anti-IgM et anti-kappa. Sur les autres pistes, seule la diminution du fond polyclonal est obervée. On notera également l'absence d'interférence des immuncomplexes et des anticorps modifiés avec la migration des globulines. Le pic migrant avant les gammas est un marqueur d'injection facilitant la superposition des profils.
o Exemple 8 : immunotypage d'un sérum contenant une protéine monoclonale de type lambda libre migrant en gamma sur Capillarys avec le tampon Sebia Capillarys B1B2+ (fenêtre d'acquisition 100-235s): 8 aliquotes de ce sérum ont été mélangées avec 7 anticorps modifiés par l'anhydride 1,2,4-benzènetricarboxylique (anti-IgG, -IgA, -IgM, -kappa, - lambda, -kappa libre, -lambda libre) et avec le milieu de ces anticorps (sans anticorps ; piste ELP). Par comparaison avec la piste ELP (en noir ; Figure 8), on observe clairement la disparition du pic monoclonal en gamma pour les aliquotes traitées par les anti-lambda et anti-lambda libre. Sur les autres pistes, seule la diminution du fond polyclonal est observée. On notera également l'absence d'interférence des immuncomplexes et des anticorps modifiés avec la migration des globulines. Le pic migrant avant les gammas est un marqueur d'injection facilitant la superposition des profils.
o Exemple 9 : comparaison de l'immunotypage obtenu avec un anticorps anti-human IgG modifié par l'anhydride 1,2,4-benzènetricarboxylique ou par l'acide mellitique/EDCI sur un sérum contenant une protéine monoclonale de type Gkappa migrant en gamma sur Capillarys avec le tampon Sebia Capillarys B1B2+ (fenêtre d'acquisition 100-235s): 3 aliquotes de ce sérum ont été mélangées avec 2 anti-human IgG modifiés par l'anhydride 1,2,4-benzènetricarboxylique et par l'acide mellitique/EDCI et avec le milieu de ces anticorps (sans anticorps ; piste ELP). Par comparaison avec la piste ELP (en noir ; Figure 9), on observe clairement la disparition du pic monoclonal en gamma pour les aliquotes traitées par les 2 anticorps modifiés. On notera également dans les 2 cas l'absence d'interférence des immuncomplexes et des anticorps modifiés avec la migration des globulines. Le pic migrant avant les gammas est un marqueur d'injection facilitant la superposition des profils.
o Exemple 10: immunotypage in-capillaire d'un sérum contenant une protéine monoclonale de type G kappa migrant en gamma sur Capillarys avec le tampon Sebia Capillarys B1B2+ (fenêtre d'acquisition 100-235s) : le milieu des anticorps (sans les anticorps) et 5 anticorps modifiés par l'anhydride 1,2,4-benzènetricarboxylique (anti-IgG, -IgA, - IgM, -kappa, -lambda) ont été placés dans une 1 ère barrette, 6 aliquotes de ce sérum ont été diluées dans le tampon Sebia Capillarys B1B2+ contenant le marqueur d'injection (barrette 2). Les anticorps puis les aliquotes de sérum dilué ont été injectés et les 6 migrations réalisées. Par comparaison avec la piste ELP (en noir; Figure 10), on observe clairement la disparition du pic monoclonal en gamma pour les aliquotes traitées par les anti-IgG et anti-kappa. Sur les autres pistes, seule la diminution du fond polyclonal est observée. On notera également l'absence d'interférence des immuncomplexes et des anticorps modifiés avec la migration des globulines. Le pic migrant avant les gammas est un marqueur d'injection facilitant la superposition des profils.
o Exemple 11 : Utilisation de l'antisérum Pentavalent (mélange d'anticorps anti-IgG, -IgA, -IgM, -kappa et -lambda modifiés par l'anhydride 1,2,4-benzènetricarboxylique) afin de vérifier la nature Mc d'un pic épaulé sur bêta-1 dans un sérum par analyse sur Capillarys avec le tampon Sebia Capillarys B1B2+ (fenêtre d'acquisition : 100-235 s) : 2 aliquotes de ce sérum ont été mélangées avec l'antisérum Pentavalent et avec le milieu de ces anticorps (sans anticorps ; piste ELP). Par comparaison avec le piste ELP (en noire ; Figure 11) , on observe clairement la disparition du pic épaulé sur bêta-1 sur la piste traitée avec l'antisérum Pentavalent, indiquant la nature Mc de ce pic (protéine Mc de type M kappa). On notera également la disparition du fond polyclonal, l'absence de fraction bêta-2 pour ce sérum ainsi que l'absence d'interférence des immuncomplexes et des anticorps modifiés avec la migration des globulines. Le pic migrant avant les gammas est un marqueur d'injection facilitant la superposition des profils.
o Exemple 12 : Immunotypage d'un sérum contenant une protéine Mc de type lamba libre migrant en gamma sur Capillarys avec le tampon Sebia Capillarys B1B2+ (fenêtre d'acquisition 100-235s) : 6 aliquotes de ce sérum ont été mélangées avec 5 anticorps modifiés par l'anhydride 1,2,4-benzènetricarboxylique (antisérum Trivalent (mélange d'anticorps anti-IgG, -IgA, -IgM), anti-kappa, anti-lambda, anti-kappa libre, anti-lambda libre) et avec le milieu de ces anticorps (sans anticorps ; piste ELP). Par comparaison avec la piste ELP (en noir; Figure 12), on observe clairement la disparition du fond polyclonal, mais pas du pic Mc sur la piste traitée avec l'antisérum trivalent. On observe en parallèle la disparition du pic Mc sur les pistes anti-lambda et anti-lambda libre. Sur les pistes anti-kappa et anti-kappa libre, seule la diminution du fond polyclonal est observée. On notera également l'absence de fraction bêta-2 pour ce sérum ainsi que l'absence d'interférence des immuncomplexes et des anticorps modifiés avec la migration des globulines. Le pic migrant avant les gammas est un marqueur d'injection facilitant la superposition des profils.
o Exemple 13 : Immunotypage d'un sérum contenant une protéine Mc de type M kappa migrant en gamma sur Capillarys avec le tampon Sebia Capillarys Protein(e) (fenêtre d'acquisition 110-275s) : 6 aliquotes de ce sérum ont été mélangées avec 5 anticorps modifiés par l'anhydride 1,2,4-benzènetricarboxylique (anti-IgG, -IgA, -IgM, -kappa, -lambda) et avec le milieu de ces anticorps (sans anticorps ; piste ELP). Par comparaison avec la piste ELP (en noire ; Figure 13), on observe clairement la disparition du pic Mc en gamma sur les aliquotes traitées par les anti-IgM et anti-kappa. Sur les autres pistes, seule la diminution du fond polyclonal est observée. On notera également l'absence d'interférence des immuncomplexes et des anticorps modifiés avec la migration des globulines. Le pic migrant avant les gammas est un marqueur d'injection facilitant la superposition des profils.

## Revendications

1. Méthode pour l'analyse électrophorétique capillaire d'un échantillon biologique, comprenant la mise en oeuvre d'anticorps modifiés surchargés négativement de façon telle qu'ils migrent en une zone située en dehors de la zone de migration des globulines de l'échantillon biologique lorsqu'elles sont séparées lors de l'électrophorèse, lesdits anticorps ayant une spécificité antigénique pour des protéines cibles monoclonales déterminées et étant obtenus par réaction avec (i) l'acide mellitique, en présence d'EDCI (1(3-diméthylaminopropyl)-3-éthylcarbodiimide hydrochloride) ou (ii) un anhydride comprenant au moins une fonction acide carboxylique.

2. Méthode selon la revendication 1, pour l'analyse électrophorétique capillaire d'un échantillon biologique, comprenant les étapes suivantes :
a) séparation des constituants d'une première partie aliquote de l'échantillon biologique par électrophorèse capillaire, en présence d'anticorps modifiés surchargés négativement ayant une spécificité antigénique déterminée et susceptibles de former un complexe de type antigène-anticorps avec une protéine monoclonale éventuellement présente dans l'échantillon biologique et détection des constituants séparés de l'échantillon biologique ;
b) comparaison du profil électrophorétique obtenu à l'étape a) avec un profil électrophorétique des constituants d'une autre partie aliquote du même échantillon séparé par électrophorèse capillaire en l'absence desdits anticorps modifiés surchargés négativement de spécificité antigénique déterminée.

3. Méthode selon la revendication 1 ou la revendication 2 dans laquelle on met en oeuvre des anticorps modifiés surchargés négativement, formant une gamme d'anticorps ayant des spécificités antigéniques différentes, mis au contact d'une même partie aliquote de l'échantillon biologique, pour réaliser la séparation par électrophorèse capillaire des constituants de l'échantillon.

4. Méthode selon la revendication 3 dans laquelle chaque type d'anticorps de ladite gamme d'anticorps a une spécificité antigénique déterminée pour un isotype d'immunoglogulines.

5. Méthode selon l'une quelconque des revendications 2 à 4, pour l'analyse électrophorétique capillaire d'un échantillon biologique divisé en au moins 2 parties aliquotes, comprenant les étapes suivantes, préalables à la séparation des constituants de l'échantillon biologique par électrophorèse
1) Mise en contact d'un milieu contenant des anticorps modifiés surchargés négativement ayant une spécificité antigénique déterminée avec une partie aliquote de l'échantillon biologique, dans des conditions d'incubation permettant la réaction immunologique entre lesdits anticorps et une protéine monoclonale cible spécifiquement reconnue par lesdits anticorps, lorsqu'elle est présente dans l'échantillon biologique, et mise en contact d'une autre partie aliquote de l'échantillon biologique avec le susdit milieu cependant dépourvu des anticorps modifiés.
2) injection dans le capillaire d'électrophorèse, des parties aliquotes de l'échantillon biologique incubées selon l'étape 1).

6. Méthode selon l'une quelconque des revendications 1 ou 2 pour l'analyse électrophorétique capillaire d'un échantillon biologique, comprenant les étapes suivantes, préalables à la séparation des constituants de l'échantillon biologique par électrophorèse :
1) injection d'un milieu contenant des anticorps modifiés surchargés négativement ayant une spécificité antigénique déterminée, dans le capillaire d'électrophorèse, et injection dudit milieu cependant dépourvu desdits anticorps modifiés dans un autre capillaire d'électrophorèse ;
2) injection de l'échantillon biologique dans les capillaires d'électrophorèse traités selon l'étape 1), dans des conditions permettant la réaction immunologique entre les anticorps et la ou les protéine(s) monoclonale(s) cible(s) lorsqu'elle(s) est(sont) présente(s) dans l'échantillon biologique.

7. Méthode selon l'une quelconque des revendications 1 à 6, dans laquelle l'échantillon biologique est divisé en au moins n+1 parties aliquotes lorsque la gamme d'anticorps modifiés surchargés négativement comprend n types d'anticorps de spécificités différentes déterminées.

8. Méthode selon l'une quelconque des revendications 1 à 7, **caractérisée en ce que** les anticorps sont choisis parmi les anticorps anti-IgG, anti-IgA, anti-IgM, anti-Kappa et anti-Lambda.

9. Méthode selon l'une quelconque des revendications 1 à 8, **caractérisée en ce que** l'on met en oeuvre des anticorps anti-IgG, des anticorps anti-IgA, des anticorps anti-IgM, des anticorps anti-Kappa, des anticorps anti-Lambda, des anticorps anti-kappa libre et des anticorps anti-lambda libre.

10. Méthode selon l'une quelconque des revendications 1 à 9, dans laquelle l'anticorps modifié surchargé négativement est en excès par rapport à la ou les protéine(s) monoclonales cible(s) qu'il reconnaît lorsqu'elle est(sont) présente(s) dans l'échantillon biologique.

11. Méthode selon l'une quelconque des revendications 1 à 10, dans laquelle les anticorps modifiés surchargés négativement sont le produit de la réaction avec l'anhydride 1,2,4-benzène tricarboxylique.

12. Méthode selon l'une quelconque des revendications 1 à 11 dans laquelle l'anticorps surchargé négativement est le produit de la réaction de l'anticorps avec un anhydride comportant au moins une fonction acide carboxylique, ladite réaction comprenant la dissolution extemporanée de l'anhydride dans du diméthylformamide (DMF) et la mise en contact de l'anhydride dissous avec l'anticorps en solution pour permettre la réaction de l'anhydride et de l'anticorps, ladite réaction étant effectuée à 37°C dans des conditions permettant le greffage de charges négatives sur l'anticorps.

13. Méthode selon l'une quelconque des revendications 1 à 12, dans laquelle l'électrophorèse capillaire est réalisée dans un tampon alcalin, à pH compris entre 9 et 11, contenant un composé tampon et au moins un additif susceptible d'augmenter la force ionique du tampon d'analyse.

14. Méthode selon l'une quelconque des revendications 1 à 13, dans laquelle l'échantillon biologique est choisi parmi des échantillons de plasma, d'urine ou de liquide céphalo-rachidien.

15. Méthode selon l'une quelconque des revendications 1 à 13, dans laquelle l'échantillon biologique est un échantillon de sérum.

16. Méthode pour la recherche et le typage de protéines monoclonales dites protéines Mc dans un échantillon biologique, comprenant l'analyse électrophorétique capillaire selon l'une quelconque des revendications 1 à 15 de différentes parties aliquotes de l'échantillon biologique, chacune des parties aliquotes étant mise au contact d'anticorps choisis respectivement parmi les immunoglobulines anti-IgG, anti-IgA, anti-IgM, anti-Kappa, anti-Lambda, anti-kappa libre et anti-lambda libre modifiées pour être surchargées négativement.

17. Méthode pour la recherche et le typage de protéines monoclonales (protéines Mc) dans un échantillon biologique, dans laquelle la présence d'une protéine Mc dans l'échantillon biologique est détectée par immunosoustraction du pic correspondant à ladite protéine Mc, dans le profil électrophorétique des protéines de l'échantillon biologique, la migration du complexe anticorps modifié surchargé négativement - protéine Mc s'effectuant en dehors du profil électrophorétique des globulines de l'échantillon biologique, ledit anticorps surchargé négativement étant obtenu par réaction avec (i) l'acide mellitique, en présence d'EDCI (1(3-diméthylaminopropyl)-3-éthylcarbodiimide hydrochloride) ou (ii) un anhydride comprenant au moins une fonction acide carboxylique.

18. Procédé pour la préparation d'anticorps modifiés surchargés négativement, comprenant les étapes de :
- dissolution extemporanée d'un anhydride comportant au moins une fonction acide carboxylique dans du diméthylformamide (DMF),
- mise en contact des anticorps avec l'anhydride dissous dans des conditions permettant la réaction de l'anhydride et de l'anticorps afin d'obtenir un anticorps modifié surchargé négativement à pH alcalin.

19. Procédé selon la revendication 18, dans lequel l'anhydride est l'anhydride benzène tricarboxylique.

20. Procédé selon l'une quelconque des revendications 18 ou 19 dans lequel l'anhydride est dissous extemporanément dans du DMF pur.

21. Anticorps modifiés surchargés négativement **caractérisés en ce que** des fonctions acide sont greffées sur les anticorps de façon à leur conférer une capacité de migration électrophorétique en dehors du profil de migration des immunoglobulines de type IgG, IgA, IgM, IgE et IgD séparées dans les mêmes conditions de séparation par électrophorèse capillaire, lesdites fonctions acide étant greffées sur lesdits anticorps par réaction avec (i) l'acide mellitique, en présence d'EDCI (1 (3-diméthylaminopropyl)-3-éthylcarbodiimide hydrochloride) ou (ii) un anhydride comprenant au moins une fonction acide carboxylique.

22. Anticorps modifiés surchargés négativement selon la revendication 21, tels qu'obtenus par mise en oeuvre d'un procédé selon l'une quelconque des revendications 18 à 20.

23. Anticorps modifiés surchargés négativement selon l'une quelconque des revendications 21 ou 22, choisis parmi les immunoglobulines anti-IgG, anti-IgA, anti-IgM, anti-Kappa, anti-Lambda, anti-kappa libre et anti-lambda libre.

24. Anticorps modifiés surchargés négativement selon l'une quelconque des revendications 21 ou 22, mélangés sous la forme d'un antisérum pentavalent contenant lesdits anticorps modifiés surchargés négativement anti-IgG, anti-IgA, anti-IgM, anti-Kappa et anti-Lambda, ou sous la forme d'un antisérum trivalent contenant lesdits anticorps surchargés négativement anti-IgG, anti-IgA et anti-IgM.

25. Anticorps modifiés surchargés négativement selon l'une quelconque des revendications 21 à 24 **caractérisés en ce qu'**il s'agit d'anticorps polyclonaux.

26. Trousse pour la détection et le typage de protéines monoclonales dans un échantillon biologique, comprenant des anticorps modifiés surchargés négativement selon l'une quelconque des revendications 21 à 25, et un diluant approprié.

27. Utilisation d'anticorps selon l'une quelconque des revendications 21 à 25, pour la détection par électrophorèse capillaire et immunodéplacement de protéines monoclonales présentes dans un échantillon biologique.

28. Utilisation d'anticorps selon l'une quelconque des revendications 21 à 25, pour le typage de protéines monoclonales présentes dans un échantillon biologique.

## Claims

1. A method for capillary electrophoretic analysis of a biological sample, comprising the use of modified antibodies negatively surcharged in such a way that they migrate into a zone located outside the migration zone for the globulins of the biological sample when they are separated during electrophoresis, said antibodies having an antigenic specificity for predetermined target monoclonal proteins and being obtained by reaction with (i) mellitic acid in the presence of EDCI (1(3-dimethylaminopropyl)-3-ethylcarbodiimide hydrochloride) or (ii) an anhydride comprising at least one carboxylic acid function.

2. A method according to claim 1, for the capillary electrophoretic analysis of a biological sample, comprising the following steps:
a) separating the constituents of a first aliquot portion of the biological sample by capillary electrophoresis in the presence of negatively surcharged modified antibodies having a predetermined antigenic specificity and which are capable of forming an antigen-antibody type complex with a monoclonal protein which may be present in a biological sample, and detecting the separated constituents of the biological sample;
b) comparing the electrophoretic profile obtained in step a) with an electrophoretic profile of the constituents of another aliquot portion of the same sample separated by capillary electrophoresis in the absence of said negatively surcharged modified antibodies with a predetermined antigenic specificity.

3. A method according to claim 1 or claim 2, in which negatively surcharged modified antibodies are used to form an array of antibodies having different antigenic specificities, brought into contact with the same aliquot portion of the biological sample, to separate the constituents of the sample by capillary electrophoresis.

4. A method according to claim 3, in which each type of antibody of said array of antibodies has a predetermined antigenic specificity for one immunoglobulin isotype.

5. A method according to any one of claims 2 to 4, for capillary electrophoretic analysis of a biological sample divided into at least 2 aliquot portions, comprising the following steps prior to separating the constituents of the biological sample by electrophoresis:
1) bringing a medium containing negatively surcharged modified antibodies with a predetermined antigenic specificity into contact with an aliquot portion of the biological sample under incubation conditions allowing the immunological reaction between said antibodies and a target monoclonal protein specifically recognized by said antibodies, when it is present in the biological sample, and bringing another aliquot portion of the biological sample into contact with said medium which is free of modified antibodies;
2) injecting the aliquot portions of the incubated biological sample of step 1) into the capillary electrophoresis.

6. A method according to claim 1 or claim 2 for capillary electrophoretic analysis of a biological sample, comprising the following steps prior to separating the constituents of the biological sample by electrophoresis:
1) injecting a medium containing negatively surcharged modified antibodies with a predetermined antigenic specificity into the electrophoresis capillary, and injecting said medium, which is free of said modified antibodies, into another electrophoresis capillary;
2) injecting the biological sample into the electrophoresis capillaries treated in accordance with step 1) under conditions allowing immunological reaction between the antibodies and the target monoclonal protein(s) when it (they) are present in the biological sample.

7. A method according to any one of claims 1 to 6, in which the biological sample is divided into at least n+1 aliquot portions when the array of negatively surcharged modified antibodies comprises n types of antibodies with different predetermined specificities.

8. A method according to any one of claims 1 to 7, **characterized in that** the antibodies are selected from anti-IgG, anti-IgA, anti-IgM, anti-kappa and anti-lambda antibodies.

9. A method according to any one of claims 1 to 8, **characterized in that** anti-IgG antibodies, anti-IgA antibodies, anti-IgM antibodies, anti-kappa antibodies, anti-lambda antibodies, free anti-kappa antibodies and free anti-lambda antibodies are used.

10. A method according to any one of claims 1 to 9, in which the negatively surcharged modified antibody is in excess with respect to the target monoclonal protein(s) which it recognizes when it (they) are present in the biological sample.

11. A method according to any one of claims 1 to 10, in which the negatively surcharged modified antibodies are the product of reaction with 1,2,4-benzene tricarboxylic anhydride.

12. A method according to any one of claims 1 to 11, in which the negatively surcharged antibody is the product of the reaction of antibody with an anhydride comprising at least one carboxylic acid function, said reaction comprising extemporaneously dissolving the anhydride in dimethylformamide (DMF) and bringing the dissolved anhydride into contact with the antibody in solution to allow the anhydride and the antibody to react, said reaction being carried out at 37°C under conditions which allow grafting of negative charges onto the antibody.

13. A method according to any one of claims 1 to 12, in which the capillary electrophoresis is carried out in an alkali buffer at a pH in the range 9 to 11 containing a buffer compound and at least one additive which can increase the ionic strength of the analyzing buffer.

14. A method according to any one of claims 1 to 13, in which the biological sample is selected from plasma, urine or cephalo-rachidian liquid samples.

15. A method according to any one of claims 1 to 13, in which the biological sample is a serum sample.

16. A method for investigating and typing monoclonal proteins termed Mc proteins in a biological sample, comprising capillary electrophoretic analysis in accordance with any one of claims 1 to 15 of different aliquot portions of the biological sample, each of the aliquot portions being brought into contact with antibodies respectively selected from anti-IgG, anti-IgA, anti-IgM, anti-kappa, anti-lambda, free anti-kappa and free anti-lambda immunoglobulins modified to be negatively surcharged.

17. A method for investigating and typing monoclonal proteins (Mc proteins) in a biological sample, in which the presence of a protein Mc in the biological sample is detected by immunosubtracting the peak corresponding to said protein Mc in the electrophoretic profile of proteins of the biological sample, migration of the negatively surcharged modified antibody-Mc protein complex being carried out outside the electrophoretic profile of the globulins of the biological sample, said negatively surcharged antibody being obtained by reaction with (i) mellitic acid in the presence of EDCI (1(3-dimethylaminopropyl)-3-ethylcarbodiimide hydrochloride) or (ii) an anhydride comprising at least one carboxylic acid function.

18. A method for preparing negatively surcharged modified antibodies, comprising the steps of:
• extemporaneously dissolving an anhydride comprising at least one carboxylic acid function in dimethylformamide (DMF);
• bringing the antibodies into contact with the dissolved anhydride under conditions which allow reaction of the anhydride and antibody to obtain negatively surcharged modified antibody at an alkali pH.

19. A method according to claim 18, in which the anhydride is benzene tricarboxylic anhydride.

20. A method according to claim 18 or claim 19, in which the anhydride is extemporaneously dissolved in pure DMF.

21. Negatively surcharged modified antibodies, **characterized in that** acid functions are grafted onto the antibodies to endow them with a capacity for electrophoretic migration outside the migration profile of type IgG, IgA, IgM, IgE and IgD immunoglobulins separated under the same capillary electrophoresis separation conditions, said acid functions being grafted onto said antibodies by reaction with (i) mellitic acid in the presence of EDCI (1(3-dimethylaminopropyl)-3-ethylcarbodiimide hydrochloride) or (ii) an anhydride comprising at least one carboxylic acid function.

22. Negatively surcharged modified antibodies according to claim 21, obtained by carrying out a method according to any one of claims 18 to 20.

23. Negatively surcharged modified antibodies according to claim 21 or claim 22, selected from anti-IgG, anti-IgA, anti-IgM, anti-kappa, anti-lambda, free anti-kappa and free anti-lambda immunoglobulins.

24. Negatively surcharged modified antibodies according to claim 21 or claim 22, mixed in the form of a pentavalent antiserum containing said negatively surcharged modified anti-IgG, anti-IgA, anti-IgM, anti-kappa and anti-lambda antibodies, or in the form of a trivalent antiserum containing said negatively surcharged anti-IgG, anti-IgA and anti-IgM antibodies.

25. Negatively surcharged modified antibodies according to any one of claims 21 to 24, **characterized in that** they are polyclonal antibodies.

26. A kit for detecting and typing monoclonal proteins in a biological sample, comprising negatively surcharged modified antibodies according to any one of claims 21 to 25, and a suitable diluent.

27. Use of antibodies according to any one of claims 21 to 25, to detect monoclonal proteins present in a biological sample by capillary electrophoresis and immunodisplacement.

28. Use of antibodies according to any one of claims 21 to 25, to type monoclonal proteins present in a biological sample.

## Patentansprüche

1. Verfahren zur Kapillarelektrophorese-Analyse einer biologischen Probe, umfassend das Einsetzen modifizierter Antikörper, welche derart negativ überladen sind, dass sie in einen Bereich wandern, welcher außerhalb des Migrationsbereiches von Globulinen der biologischen Probe liegt, wenn sie während der Elektrophorese separiert werden, wobei diese Antikörper eine antigene Spezifität für definierte monoklonale Zielproteine aufweisen und durch Reaktion mit (i) Mellitsäure in Gegenwart von EDCl (1(3-Dimethylaminopropyl)-3-ethylcarbodiimidhydrochlorid) oder (ii) einem Anhydrid, das wenigstens eine Carbonsäurefunktion umfasst, erhalten worden sind.

2. Verfahren gemäß Anspruch 1, zur Kapillarelektrophorese-Analyse einer biologischen Probe, umfassend die folgenden Stufen:
a) Abtrennen von Bestandteilen einer ersten aliquoten Partie der biologischen Probe durch Kapillarelektrophorese, in Gegenwart von modifizierten, negativ überladenen Antikörpern, die eine definierte antigene Spezifität aufweisen und einen Komplex vom Typ Antigen-Antikörper mit einem gegebenenfalls in der biologischen Probe vorliegenden monoklonalen Protein bilden können und Erfassen von von der biologischen Probe abgetrennten Bestandteilen;
b) Vergleichen des in Stufe a) erhaltenen Elektrophoreseprofils mit einem Elektrophoreseprofil von Bestandteilen einer anderen aliquoten Partie der selben Probe, abgetrennt durch Kapillarelektrophorese in Abwesenheit dieser modifizierten, negativ überladenen Antikörper definierter antigener Spezifität.

3. Verfahren gemäß Anspruch 1 oder Anspruch 2, worin man modifizierte, negativ überladene Antikörper einsetzt, welche ein Band von Antikörpern mit verschiedenen antigenen Spezifitäten aufweist, man eine gleiche aliquote Partie der biologischen Probe in Kontakt bringt, um die Abtrennung von Bestandteilen der Probe durch Kapillarelektrophorese durchzuführen.

4. Verfahren gemäß Anspruch 3, worin jede Art von Antikörpern dieses Bandes von Antikörpern eine definierte antigene Spezifität für einen Isotyp von Immunoglobulinen besitzt.

5. Verfahren gemäß einem beliebigen der Patentansprüche 2 bis 4, zur Kapillarelektrophorese-Analyse einer in wenigstens zwei aliquote Partien geteilten biologischer Probe, welche vor der Abtrennung von Bestandteilen der biologischen Probe durch Elektrophorese folgende Stufen umfasst:
1) in Kontakt bringen eines Milieus, welches modifizierte, negativ überladene Antikörper mit einer definierten antigenen Spezifität enthält, mit einer aliquoten Partie der biologischen Probe, unter Inkubationsbedingungen, welche die immunologische Reaktion zwischen diesen Antikörpern und einem spezifisch durch diese Antikörper erkannten monoklonalen Zielprotein erlaubt, wenn es in der biologischen Probe vorhanden ist und in Kontakt bringen einer weiteren aliquoten Partie der biologischen Probe mit dem oben genannten Milieu, jedoch ohne den modifizierten Antikörpern,
2) Injizieren aliquoter Partien der gemäß Stufe 1) inkubierten biologischen Probe in die Kapillarelektrophorese.

6. Verfahren gemäß irgendeinem der Patentansprüche 1 oder 2 zur Kapillarelektrophorese-Analyse einer biologischen Probe, welche vor der Abtrennung von Bestandteilen der biologischen Probe durch Elektrophorese die folgenden Stufen umfasst:
1) Injizieren eines Milieus, welches modifizierte, negativ überladene Antiköper mit einer definierten antigenen Spezifität besitzt, in die Elektrophoresekapillare und injizieren dieses Milieus, jedoch ohne diesen modifizierten Antikörpern, in eine andere Elektrophoresekapillare;
2) Injizieren der gemäß Stufe 1) behandelten Probe in die Elektrophoresekapillaren, unter Bedingungen, welche die immunologische Reaktion zwischen den Antikörpern und dem oder den monoklonalen Zielprotein(en) erlaubt, wenn es oder sie in der biologischen Probe vorliegt bzw. vorliegen.

7. Verfahren gemäß irgendeinem der Ansprüche 1 bis 6, worin die biologische Probe in wenigstens n+1 aliquote Partien geteilt wird, wenn das Band modifizierter, negativ überladener Antikörper n-Typen von Antikörpern von definierten verschiedenen Spezifitäten umfasst.

8. Verfahren gemäß irgendeinem der Ansprüche 1 bis 7, **dadurch gekennzeichnet, dass** die Antikörper aus den Antikörpern anti-IgG, anti-IgA, anti-IgM, anti-Kappa und anti-Lambda ausgewählt sind.

9. Verfahren gemäß irgendeinem der Ansprüche 1 bis 8, **dadurch gekennzeichnet, dass** man anti-IgG-Antikörper, anti-IgA-Antikörper, anti-IgM-Antikörper, anti-Kappa-Antikörper, freie anti-Kappa-Antikörper und freie anti-Lampda-Antikörper einsetzt.

10. Verfahren gemäß irgendeinem der Ansprüche 1 bis 9, worin die modifizierten, negativ überladenen Antikörper bezogen auf das oder die monoklonale(n) Zielprotein(e) im Überschuss vorliegen, welche er erkennt, wenn es (sie) in der biologischen Probe vorliegt (vorliegen).

11. Verfahren gemäß irgendeinem der Ansprüche 1 bis 10, worin die modifizierten, negativ überladenen Antikörper das Produkt der Umsetzung mit 1,2,4-Benzoltricarbonsäureanhydrid sind.

12. Verfahren gemäß irgendeinem der Ansprüche 1 bis 11, worin die negativ überladenen Antikörper das Produkt der Umsetzung der Antikörper mit einem Anhydrid sind, das wenigstens eine Carbonsäurefunktion aufweist, wobei die Umsetzung die vorherige Auflösung des Anhydrids in Dimethylformamid (DMF) und das in Kontakt bringen des aufgelösten Antikörpers mit in Lösung befindlichen Antikörpern umfasst, um die Reaktion des Anhydrids und der Antikörper zu gestatten, wobei die Reaktion bei 37°C unter Bedingungen durchgeführt wird, welche das Aufpfropfen negativer Ladung auf die Antikörper erlaubt.

13. Verfahren gemäß irgendeinem der Ansprüche 1 bis 12, worin die Kapillarelektrophorese in einem alkalischen Puffer mit einem zwischen 9 und 11 enthaltenen pH-Wert durchgeführt wird, der eine Pufferverbindung und wenigstens ein Additiv enthält, das die Ionenstärke des Analysepuffers steigern kann.

14. Verfahren gemäß irgendeinem der Ansprüche 1 bis 13, worin die biologische Probe unter Proben von Plasma, Urin oder Cephalo-Rachidien-Flüssigkeit ausgewählt ist.

15. Verfahren gemäß irgendeinem der Ansprüche 1 bis 13, worin die biologische Probe eine Serumprobe ist.

16. Verfahren zur Untersuchung und Typifizierung von Mc-Proteine genannten monoklonalen Proteinen in einer biologischen Probe, umfassend die Kapillarelektrophorese-Analyse gemäß irgendeinem der Ansprüche 1 bis 15 von verschiedenen aliquoten Partien der biologischen Probe, wobei jede der aliquoten Partien in Kontakt mit Antikörpern gebracht werden, die jeweils unter den anti-IgG-, anti-IgA-, anti-IgM-, anti-Kappa-, anti-Lampda-, freien anti-Kappa- und freien anti-Lampda-Immunoglobulinen ausgewählt sind, die modifiziert sind, um negativ überladen zu sein.

17. Verfahren zur Untersuchung und Typifizierung von monoklonalen Proteinen (Mc-Proteinen) in einer biologischen Probe, worin die Gegenwart eines Mc-Proteins in der biologischen Probe durch Immunosubtraktion des dem Mc-Protein entsprechenden Peaks im elektrophoretischen Profil des Proteins der biologischen Probe, der Migration des Komplexes aus modifizierte, negativ überladene Antikörper und Mc-Protein, welche außerhalb des elektrophoretischen Profils der Globuline der biologischen Probe auftreten, festgestellt wird, wobei diese negativ überladenen Antikörper durch Reaktion mit (i) Mellitsäure in Gegenwart von EDCl (1(3-Dimethylamino-propyl)-3-ethylcarbodiimidhydrochlorid) oder (ii) einem Anhydrid, das wenigstens eine Carbonsäurefunktion umfasst, erhalten worden sind.

18. Verfahren zur Herstellung modifizierter, negativ überladener Antikörper, umfassend die Stufen:
- vorherige Auflösung eines Anhydrids, das wenigstens eine Carbonsäurefunktion umfasst, in Dimethylformamid (DMF),
- in Kontakt bringen der Antikörper mit aufgelöstem Anhydrid unter Bedingungen, welche die Reaktion des Anhydrids und der Antikörper erlaubt, um modifizierte, negativ überladene Antikörper mit alkalischem pH-Wert zu erhalten.

19. Verfahren gemäß Anspruch 18, worin das Anhydrid Benzoltricarbonsäureanhydrid ist.

20. Verfahren gemäß irgendeinem der Ansprüche 18 oder 19, worin das Anhydrid vorher in reinem DMF gelöst wird.

21. Modifizierte, negativ überladene Antikörper, **dadurch gekennzeichnet, dass** Säurefunktionen auf die Antikörper auf gepfropft sind, um ihnen die Fähigkeit zur elektrophoretischen Migration außerhalb des Migrationsprofils von Immunoglobulinen vom Typ IgG, IgA, IgM, IgE und IgD zu verleihen, getrennt unter denselben Auftrennungsbedingungen durch Kapillarelektrophorese, wobei diese Säurefunktionen auf diese Antikörper durch Umsetzung mit (i) Mellitsäure in Gegenwart von EDC1 (1(3-Dimethylaminopropyl)-3-ethylcarbodiimidhydrochlorid) oder (ii) einem Anhydrid, das wenigstens eine Carbonsäurefunktion umfasst, aufgepfropft werden.

22. Modifizierte, negativ überladene Antikörper gemäß Anspruch 21, wie sie durch den Einsatz eines Verfahrens gemäß irgendeinem der Ansprüche 18 bis 20 erhalten werden.

23. Modifizierte, negativ überladene Antikörper gemäß irgendeinem der Ansprüche 21 oder 22, ausgewählt unter anti-IgG-, anti-IgA-, anti-IgM-, anti-Kappa-, anti-Lambda-, freien anti-Kappa- und freien anti-Lambda-Immunoglobulinen.

24. Modifizierte, negativ überladene Antikörper gemäß irgendeinem der Ansprüche 21 oder 22, vermischt in Form eines fünfwertigen Antiserums, welches die modifizierten, negativ überladenen Antikörper anti-IgG, anti-IgA, anti-IgM, anti-Kappa und anti-Lambda enthält oder in Form eines dreiwertigen Antiserums, welches die negativ überladenen Antikörper anti-IgG, anti-IgA und anti-IgM enthält.

25. Modifizierte, negativ überladene Antikörper gemäß irgendeinem der Ansprüche 21 bis 24, **dadurch gekennzeichnet, dass** es sich um polyklonale Antikörper handelt.

26. Set zur Feststellung und Typifizierung von monoklonalen Proteinen in einer biologischen Probe, umfassend modifizierte, negativ überladene Antikörper gemäß irgendeinem der Ansprüche 21 bis 25 und ein geeignetes Verdünnungsmittel.

27. Verwendung der gemäß irgendeinem der Ansprüche 21 bis 25 erhaltenen Antikörper zur Feststellung von in einer biologischen Probe vorliegenden monoklonalen Proteinen durch Kapillarelektrophorese und Immunoverschiebung.

28. Verwendung von Antikörpern gemäß irgendeinem der Ansprüche 21 bis 25 zur Typifizierung von in einer biologischen Probe vorhandenen monoklonalen Proteinen.
